# EUROPÄISCHE PATENTSCHRIFT

(11) **EP 1 850 898 B1**
(45) Veröffentlichungstag und Bekanntmachung des Hinweises auf die Patenterteilung: **10.03.2021**
(21) Anmeldenummer: 06705970.9
(22) Anmeldetag: 10.02.2006
(51) Int. Cl.: A61M 16/00, A61B 5/087

(54) **VORRICHTUNG ZUR BEATMUNG**
RESPIRATORY EQUIPMENT
DISPOSITIF DE VENTILATION

(30) Priorität: 10.02.2005 DE 102005006103
(43) Veröffentlichungstag der Anmeldung: 07.11.2007
(73) Patentinhaber: Löwenstein Medical Technology S.A., 2557 Luxembourg (LU)
(72) Erfinder: PLATTNER, Diana, 76137 Karlsruhe (DE); SCHÖLLER, Bernd, 76135 Karlsruhe (DE); SCHWAIBOLD, Matthias, 76137 Karlsruhe (DE)
(74) Vertreter: Klickow & Wetzel PartGmbB
(86) Internationale Anmeldenummer: PCT/DE2006/000253
(87) Internationale Veröffentlichungsnummer: WO 2006/084456

(56) Entgegenhaltungen:
- EP-A- 1 136 094
- DE-A1- 10 118 475
- US-A- 6 000 396
- US-A1- 2002 088 465
- US-A1- 2002 185 130
- US-B1- 6 530 372

## Beschreibung

Die Erfindung betrifft eine Vorrichtung zur Beatmung, die eine mit einem Patienteninterface verbindbare Atemgasquelle sowie eine Steuereinrichtung aufweist, die zur Vorgabe von mindestens zwei unterschiedlichen Modi der Beatmung ausgebildet ist und bei der die Steuereinrichtung zur automatischen Ermittlung, Auswahl und Anwendung der verschiedenen Modi der Beatmung bei wenigstens einigen Atemstörungen entsprechend einem aktuellen patientenspezifischen Bedarf ausgebildet ist.

Für das Krankheitsbild des obstruktiven Schlafapnoesyndroms ist eine Ermittlung des individuellen Drucks für die CPAP-Therapie notwendig (continuous positive airway pressure, kontinuierlich positiver Atemwegsdruck). Die CPAP-Therapieeinstellung erfolgt üblicherweise während der sogenannten Titrationsnacht im Schlaflabor. Dabei werden unterschiedliche Verfahren angewendet, um den für den Patienten optimalen konstanten Druck für die spätere Beatmungstherapie zu ermitteln, der ein Offenhalten der oberen Atemwege gewährleistet. Die Therapieeinleitung ist zur Zeit recht konservativ. Sie erfolgt fast ausschließlich in der Klinik und nicht in häuslicher Umgebung. Der optimale therapeutische CPAP kann durch manuelle oder automatische Titration während einer Polysomnographie oder einer respiratorischen Polygraphie ermittelt werden.

Die übliche Titrationsmethode zur Findung des optimalen CPAP-Druckes besteht in einer schrittweise Erhöhung des CPAP-Druckes während einer nächtlichen Polysomnographie, bis obstruktive Apnoen, Hypopnoen und Schnarchen nicht mehr auftreten.

Alternativ kann ein CPAP-Gerät mit automatischer Druckregulation (Auto-CPAP-Gerät) in der Klinik und/oder beim Patienten über eine oder mehrere Nächte zu Hause angewendet werden. Eine Druckperzentile kann anschließend anhand der im Gerät aufgezeichneten Daten ermittelt und für die Langzeittherapie fix eingestellt werden, obwohl diese Geräte eigentlich die Aufgabe haben, dem Patienten den minimalsten aktuell erforderlichen Beatmungsdruck zuzuführen.

Zurzeit stellt die nasale CPAP-Therapie (nCPAP) mit konstantem Druck mit einer individuell einstellbaren Steigerungsrate zu Beginn des Schlafes die Methode der Wahl bei mittel- und schwergradigem Schlafapnoesyndrom dar. Besteht hierbei ein besonders hoher Druckbedarf, so wird dieser von den Patienten schlecht toleriert. Oft kommt hier das APAP- oder Bi-Level-Verfahren zum Einsatz. Bei letzterem wird inspiratorisch ein höherer und exspiratorisch ein niedriger Druck appliziert. Die Gesamtdruckbelastung wird dadurch geringer. Damit gelingt es, nochmals einen Teil der Patienten auf ein Überdruckverfahren einzustellen.

Viele Patienten mit obstruktiver Schlaf-Apnoe weisen einen stark schwankenden Druckbedarf auf, dabei können "Auto-CPAP-Gerät", auch "intelligente CPAP-Geräte" genannt, weiterhelfen. Sie erkennen zu jedem Zeitpunkt, ob sich eine Obstruktion entwickelt und greifen rechtzeitig steuernd, d.h. mit einer Druckerhöhung, ein, um den Verschluss der Atemwege zu verhindern.

Durch in das Messverfahren integrierte Druckoszillationen wird eine Differenzierung von sog. offenen bzw. geschlossenen Apnoen ermöglicht.

Die Variation des automatischen CPAP-Druckes erfolgt in Abhängigkeit von respiratorischen Obstruktionszeichen. Eine Reduktion des Druckes erfolgt auf minimal 3 cmH2O.

Ein weiteres Einsatzgebiet der Überdruckbeatmung ist die nichtinvasive Beatmung. Dabei unterstützt das Beatmungsgerät den insuffizienten Atemapparat. Technische Neuerungen wie handlichere Beatmungsgeräte und vor allem leckagereduzierende Nasenmasken, die in vielen Fällen die Tracheotomie ersetzen, brachten in den letzten Jahren Verbesserungen der therapeutischen Möglichkeiten.

Indikationen für die Heimbeatmung sind u. a. Ventilationsstörungen bei neuromuskulären Erkrankungen(z.B. zentrales Schlafapnoesyndrom, primäres alveoläres Hypoventilationssyndrom, Adipositas-Hypoventilationssyndrom, CPAPrefraktäres obstruktives Schlafapnoesyndrom, Poliomyelitis und Postpoliosyndrom, spinale Muskelatrophien, hohe zervikale Läsionen, Polyradikulitis Guillain-Barré, Myopathien)sowie restriktive Ventilationssyndrome bei Erkrankungen von Skelett, Pleura und Lunge(z.B. Kyphoskoliose, Status nach ausgedehnter Lungenresektion, ausgedehnte narbige Veränderungen von Pleura und Lunge, Status nach Thorakoplastik).

Bei der Titration des optimalen Therapiedrucks zur Behandlung des obstruktiven Schlafapnoesyndroms werden für gewöhnlich Signalgrößen gesucht, welche eine Obstruktion frühzeitig erkennen lassen. Zum Beispiel ist eine Plateaubildung des inspiratorischen Flusssignals als sensitives Zeichen einer geringgradigen Obstruktion zu beobachten. Eine Anhebung des CPAP-Druckes wirkt einer Widerstandserhöhung der oberen Luftwege entgegen und führt zur Normalisierung der Flusskontur.

Eine zuverlässige, aber sehr invasive Methode zur Erfassung des Obstruktionsniveaus stellt die kombinierte Messung des Ösophagusdruckes und des Atemflusses dar.

Eine komfortable Alternative ist die Messung der respiratorischen Impedanz.

Automatische CPAP-Titrationen basieren auf respiratorischen Kenngrößen, die den Schweregrad der Obstruktion der oberen Atemwege charakterisieren.

Die schrittweise Anpassung des CPAP-Druckes oder der Ventilationsparameter während einer nächtlichen Polysomnographie (PSG) zur optimalen Einstellung des Patienten ist nicht frei von subjektiven Kriterien. Für die Standardisierung, Reproduzierbarkeit und das Nachvollziehen der automatisierten Prozedur wäre es ein großer Vorteil, wenn mittels festgelegter Algorithmen eine ausreichend gute Beatmungseinstellung möglich wäre.

Ein weiterer Vorteil kann darin bestehen, dass nur während der Obstruktion bzw. Minderventilation der computergestützt angepasste Druck appliziert und z. B. im Wachzustand oder in Phasen nichtobstruktiver Atmung der nCPAP-Druck deutlich abgesenkt wird. Damit könnte sich auch die Akzeptanz der Methode und evtl. auch die Schlafqualität bereits während der Titration verbessern lassen.

Zur automatischen Titration können verschiedene Messgrößen wie Sauerstoffsättigung und Atemfluss in Erwägung gezogen werden. Z. B kann eine Widerstandserhöhung der oberen Luftwege u. a. zu einer Abflachung der Kontur der inspiratorischen Atemfluss/Zeitkurve führen. Aus dem Muster des inspiratorischen Atemflusses oder anderen physiologischen oder Gerätesignalen kann somit ein erhöhter Widerstand im Bereich der extrathorakalen Atemwege bereits vor Auftreten relevanter Hypopnoen erkannt werden.

Aus der US 6,530,372 B1 ist bereits ein Beatmungsgerät mit einer Einrichtung zum automatischen Bestimmen eines kritischen Luftdruckes bekannt.

Die US 6,000,396 A beschreibt ein Beatmungsgerät mit einem elektronischen Beatmungssystem. Das Gerät kann in unterschiedlichen Modi betrieben werden.

Die US 2002/0088465 A1 beschreibt ein Beatmungsgerät, bei dem die Ermittlung eines maximalen Flusses möglich ist.

Aus der US 2002/0185130 A1 ist ein Beatmungsgerät mit einer Einrichtung zur automatischen Erfassung von kritischen Gesundheitszuständen des Patienten bekannt.

Die DE 101 18 475 A1 beschreibt ein Beatmungsgerät mit einer selbstregulierenden Druckregelung.

Aus der EP 1 136 094 A2 ist ein Beatmungsgerät mit zwei unterschiedlich einstellbaren Druckpegeln bekannt. Die Steuerung des Beatmungsdruckes erfolgt in Abhängigkeit von einem gemessenen Beatmungsparameter unter Verwendung einer Mustererkennung.
Aufgabe der vorliegenden Erfindung ist es eine Vorrichtung der einleitend genannten Art derart zu konstruieren, dass eine automatische Titration unterstützt wird.

Diese Aufgabe wird erfindungsgemäß durch die Merkmale von Patentanspruch 1 gelöst.

Somit kann die Einstellung auch unabhängig von der Räumlichkeit, z.B. beim Patienten zu Hause durchgeführt werden. Darüber hinaus kann das Gerät über eine Datenverarbeitungsprogramm zur Ferneinstellung, Therapiekontrolle und Autotitration genutzt werden, wobei dieses eine automatische Detektion des angeschlossenen Gerätes beim Kommunikationsaufbau durchführt und sämtliche Titrationsparameter eingestellt werden können.

Hierdurch können in erheblichem Umfang erheblich Kosten und Zeit gespart werden, da mehr Patienten mit weniger Personal gleichzeitig überwacht werden können. Zudem können in der Heimeinstellung bessere Ergebnisse durch die gewohnte Umgebung des Patienten erzielt werden.

Durch ein solches Titrations-/Therapiegerät kann ferner die bestehende Lücke zwischen der Diagnose und Therapie geschlossen werden.

Weitere Einzelheiten der Erfindung ergeben sich aus der nachfolgenden Beschreibung in Verbindung mit der Zeichnung. Es zeigen:
- Fig. 1: eine Bedienmaske zur Eingabe und Anzeige von Geräteparametern,
- Fig. 2: eine weitere Bedienmaske zur Ereignisbewertung,
- Fig. 3: eine Darstellung von kumulierten Druckverläufen,
- Fig. 4: ein gerätetechnisches Strukturbild,
- Fig. 5: eine Darstellung eines angezeigten Druckverlaufes,
- Fig. 6: eine Bildschirmanzeige einer Ergebniszusammenstellung,
- Fig. 7: Signalverläufe zur Durchführung der Titration,
- Fig. 8: eine perspektivische Darstellung eines Beatmungsgerätes mit Atemgasschlauch und Atemmaske,
- Fig. 9: ein Druckverlauf zur Veranschaulichung einer Steuerungscharakteristik des Beatmungsgerätes,
- Fig. 10: ein Ablaufdiagramm zur Durchführung der Gerätesteuerung und
- Fig. 11: ein Blockdiagramm zur strukturierten Darstellung der Gerätesteuerung.

Das Verfahren sieht eine Analyse durch zumindest einen Sensor vor, welcher physiologische sowie gerätespezifische Signale wie zum Beispiel Compliance, Resistance, Blutwerte (O2, CO2, HB, CO, etc.), Druck, Flow, MDS, Temperatur, Motorleistung, Lüfterstrom und -spannung aufnimmt und an einen Analysator übermittelt. Über eine Auswahlfunktion gleicht der Analysator die erhaltenen Signalinformationen mit denen in einem Speicher abgelegten Informationen (löschbar, änderbar) ab. Die Informationen basieren dabei auf einer individuellen Patientenhistorie und/oder aus ausgewerteten Patientenhistorien, welche mittels Fuzzy-Logik und/oder neuronalen Netzwerken miteinander verknüpft und ausgewertet werden. Der Analysator tätig eine Moduszuordnung und entsprechend der Moduszuordnung inklusive Parametereinstellung wird eine Steuereinheit derart aktiviert, dass Effektoren (Anzeige, Gebläse, etc.) angesteuert werden.

Alternativ [spez. für APAP-Titration und BiLevel-Titration] kann die Analyse auch durch eine Person erfolgen, welche die Moduszuordnung selbstständig tätigt.

Das Konzept sieht in einer besonders bevorzugten Form vor, dass in der Diagnosephase der Arzt eine bestehende Beatmungsform/-art (Automatisch mit fest eingestellten Druckprofilen, CPAP, APAP, BiLevel, Autotitration CPAP, Autotitration BiLevel, etc.) wählen und einstellen kann. Es kann aber auch eine automatische Option gewählt werden, in dem das Gerät selbstständig an Hand verschiedener vom Patienten gelieferter und gemessener Parameter die Beatmung beginnt.

In den entsprechenden Modi wird nach der Titrationsphase durch das Gerät eine Therapieempfehlung ausgegeben, welche entweder gleich oder ohne erneute Rücksprache mit dem Arzt, umgesetzt wird oder erst durch den Arzt/Anwender bestätigt werden muss. Darüber hinaus werden weitere Empfehlungen zu einer eventuell nötigen Sauerstoff- oder Aerosoltherapie und/oder einer zu verwendenden Maske oder Mensch-Maschine-Schnittstelle ausgegeben.

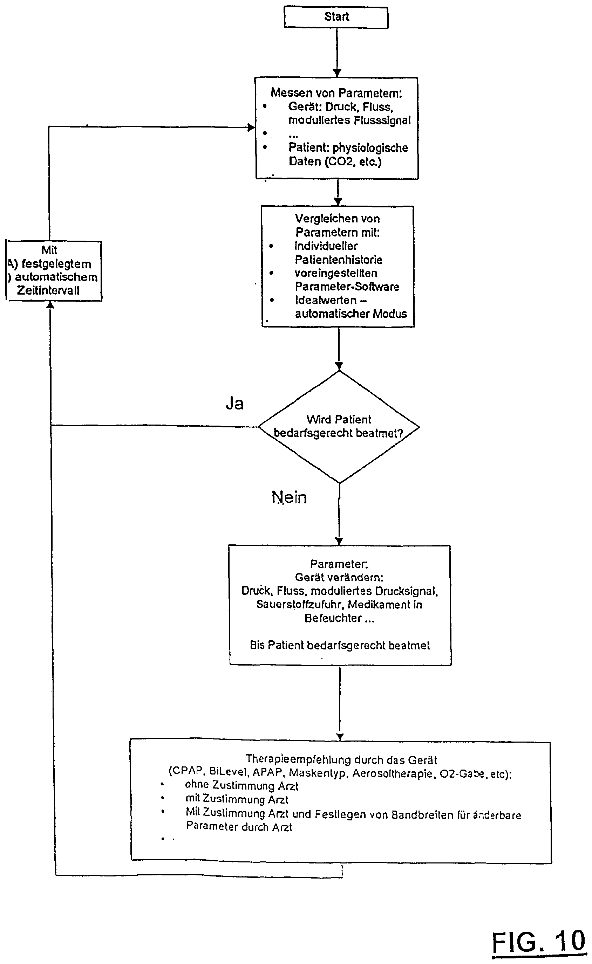

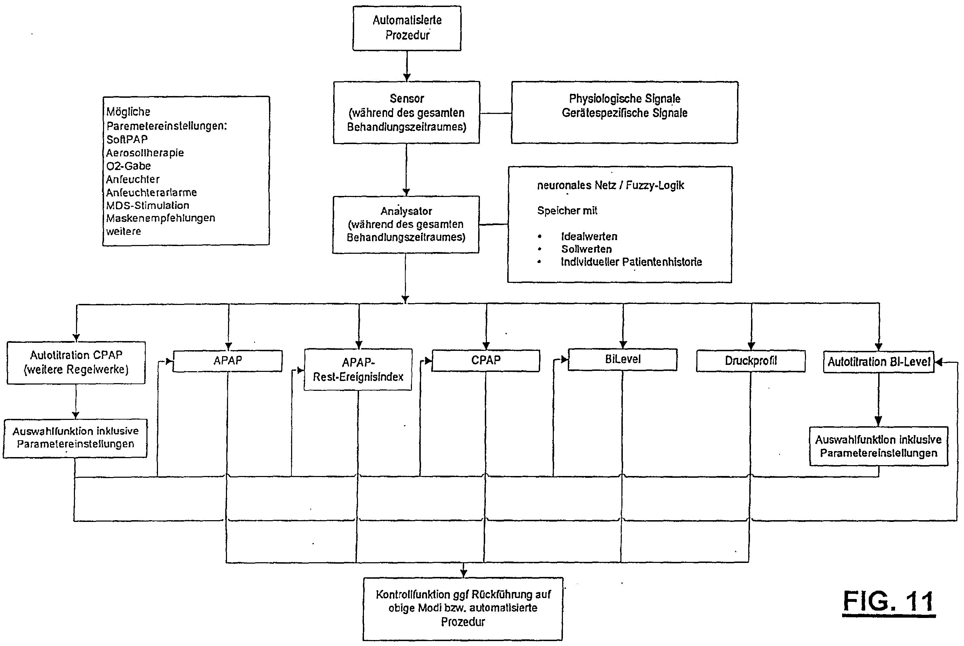

In einer weiteren besonders bevorzugten Ausführungsform werden nach dem Starten des Gerätes aktuelle Parameter des Gerätes wie z.B. Druck, Flow, MDS, Temperatur als auch des Patienten wie z.B. Compliance, Resistance, Blutwerte (O2, CO2, HB, CO, etc.) gemessen. Dies wird in Fig. 10 veranschaulicht. Diese werden in einem weiteren Schritt mit den Daten einer individuellen Patientenhistorie, in der Parameter-Software voreingestellten Werten und/oder in einem automatischen Modus mit Idealwerten abgeglichen und es wird eine Bewertung der bedarfsgerechten Beatmung des Patienten errechnet. Dies wird in Fig. 11 deutlich.

Es wird eine Empfehlung des Gerätes zur bedarfsgerechten Beatmung generiert, welche die Form der Beatmung wie z.B. CPAP, APAP, BiLevel samt der dazugehörigen Beatmungsparameter als auch die Art von zusätzlichen, unterstützenden Funktionen des Gerätes wie z.B. O2-Gabe, Monitoring und Speicherung von Patientendaten, Aerosoltherapie, Befeuchter, Vollgesichtsmaske etc. dem Arzt vorschlägt. Dieser kann daraufhin die Therapie nach Vorschlag des Gerätes akzeptieren oder die Parameter zur Beatmung in Bandbreiten, in welchen die Parameter vom Gerät selbstständig verändert werden dürfen, anpassen und einstellen.

Der Arzt kann bei einer Ersteinstellung des Gerätes diesem auch die fast vollständige Autonomie zur Änderung der Paramter übertragen, so dass nach der Therapieempfehlung das Gerät automatisch in den empfohlenen Modus springt und den Patienten in diesem Modus weiter beatmet.

Das Gerät überprüft und ändert laufend die Parameter des Gerätes bis der Patient bedarfsgerecht beatmet wird. Dabei kann entweder mit einem festgelegten oder einem automatischen Intervall gearbeitet oder nach Schwere der Atemstörung die Ausgabe einer neuen Empfehlung generiert werden.

Bei bedarfsgerechter Beatmung des Patienten kann nach einem festgelegten oder einem automatischen Intervall der Zyklus von neuem beginnen.

Das Gerät und das Verfahren nutzen eine Technologie, welche eine kontinuierliche automatische aus MDS- (moduliertes Druck Signal), flow- und druckbezogenen Größen abgeleitete Ereignisdetektion sowie beatmungsdruckrelevante Artefaktdetektion enthält, um den applizierten Beatmungsdruck zu steuern - hier jedoch nicht nur zur Steuerung eines Beatmungsdrucks, sondern auch zur Ermittlung verschiedenster für die Beatmung wichtiger Parameter. Die beim Atmen auftretenden Ereignisse und Artefakte werden in den Titrationsmodi während der Beatmungsapplikation einem ereignisindexbildenden Regelwerk zugeführt, um die für den Patienten optimalen und suffizienten Therapiedrücke u. a. Beatmungsparameter zu ermitteln.

Die APAP-Geräte sind mit Druckgasquellen ausgestattet, die über einen Beatmungsschlauch und eine Beatmungsmaske mit den Atemwegen eines Patienten verbunden sind. Eine Steuerung der Druckgasquelle erfolgt in der Regel derart, dass in Abhängigkeit von einem gemessenen Atemparameter, beispielsweise dem Flow oder dem Druck, eine spezifische Drucksteuerung vorgenommen wird.

Als ein weiteres Ausführungsbeispiel, insbesondere für die ambulante Titration und/oder für die Titration beim Anwender zu Hause, ist daran gedacht, dass ein Diagnose-Gerät physiologische Signale eines Patienten registriert und einer Auswertung zuführt. Die Auswertung erfolgt automatisch nach definierbaren Kriterien wie beispielsweise AHI.

Entsprechend dem Ergebnis der Auswertung signalisiert das Diagnose-Gerät eine Empfehlung, beispielsweise als Aufforderung an den Patienten, ein Beatmungsgerät, wie beispielsweise ein CPAP- APAP- Bilevel-Gerät, zu benutzen.

Das Diagnose-Gerät kommuniziert mit dem Beatmungsgerät im Sinne einer Titration. Beide Geräte können einander beeinflussen. Bevorzugt ist daran gedacht, dass das Diagnose-Gerät das Beatmungsgerät steuert.

Durch die Anpassung der Beatmungsparameter wird eine für den Patienten optimale Einstellung des Beatmungsgerätes ermittelt und/oder beibehalten. Wird in einer weiteren Ausführungsform eine Therapiebedürftigkeit festgestellt, löst das Diagnose-Gerät einen Alarm zum Wecken des Patienten aus, damit dieser sich das Beatmungsgerät anlegen kann.

Im zweiten Teil der Nacht zeichnet das Diagnose-Gerät weiterhin Signale auf und steuert das Beatmungsgerät, im Sinne einer Titration, derart, dass eine optimale Therapie für den Patienten ermittelt und/oder beibehalten wird.

Dadurch wird in nur einer Nacht eine Diagnose und eine Therapieeinstellung zu Hause beim Patienten ermöglicht.

In einer ergänzenden Ausführungsform erhält der Arzt einen Diagnose- und einen Therapiebericht aus derselben Nacht.

Ein weiterer großer Vorteil ist die optionale Kopplung von Therapiegerät mit Diagnosegerät bzw. mit PC-Software des Diagnosegeräts zur Konfiguration des Therapiegerätes oder der Einspeisung von Signalen oder erkannten Ereignissen oder internen Zuständen des Therapiegerätes in das Diagnosegerät. Dabei ist die Einspeisung online oder offline mit einer sehr schnellen Daten-Übertragungszeit möglich, so dass das Therapiegerät gekoppelt mit einem Gerät zur Erfassung polysomnographischer Daten eine Diagnostik bzgl. Schlafstadien und -position, Hypoxie, kardialer Störungen, Hyper/Hypokapnie, Beinbewegungen, Atemanstrengung, Mund- und Maskenleckagen, Mundexspirationen, Obstruktionszeichen wie Schnarchen, Flusslimitierungen, obstruktive Apnoen und Hypopnoen sowie Hyper- und Hypoventilation, zentral bedingte Atemstörungen u. a. vornehmen kann.

Das Gerät arbeitet sowohl standalone, als auch in Verbindung mit Computerprogrammen zur Ferneinstellung und Datenvisualisierung und -auswertung. Abhängig vom eingestellten Gerätemodus wird die ereignisabhängige Druckanpassung durchgeführt. Die Ereigniserkennung und -speicherung ist jedoch in allen Gerätemodi identisch.

Es verfügt über folgende Modi:
- Modus Autotitration CPAP (verschiedene Regelwerke stehen zur Wahl)
- Modus Druckprofil (fixe und einstellbare Druckprofile)
- Modus CPAP (für die Kontrollnacht oder manuelle Titration)
- Modus APAP
- Modus Bi-Level (optional)
- Modus APAP-Restindex
- Modus Autotitration Bi-Level (optional)
- Modus Automatisch (Gerät wählt Beatmungsmodus selbsttätig oder in Folge der vom Diagnosegerät bzw. vom Sensor und Analysator gelieferten Daten) (optional)

### Moduswahl mit Effektorenansteuerung

Im Modus Autotitration CPAP wird die Druckanpassung nach Ereignissen entsprechend des gewählten Regelwerks durchgeführt. Das Regelwerk definiert die Gewichtung der aufgetretenen Ereignisse (für die Berechnung des Ereignisindex), sowie die Parameter der Druckanpassung.

Als bevorzugte Ausführungsform sind ein festes Regelwerk (AT1) und ein benutzerdefiniertes Regelwerk (AT2) vorgesehen. Der Benutzer kann das feste bzw. benutzerdefinierte Regelwerk im Standby (Offline-) Betrieb aktivieren. Die Regelwerks-Parameter können im Standby (Offline-) Betrieb mit einer PC-Software (über die serielle Schnittstelle) angezeigt und modifiziert (nur benutzerdefiniertes Regelwerk) werden.

Dabei können vom Anwender in einer besonders bevorzugten Ausführungsform für die Beatmung im Regelwerk, welches auch über die Schnittstelle eingestellt werden kann, unter anderem der Zeitraum bzw. verschiedene Zeiträume für Titrationsbeginn, -ende, Mindestwartezeiten für Druckanpassung sowie die folgenden Parameter vorgegeben bzw. eingestellt werden:
Startdruck, Regelungsbeginn, untere und obere Druckgrenze, Druckanstiegsgeschwindigkeit, Regel für empfohlenen Titrationsdruck, Splitnightoption.

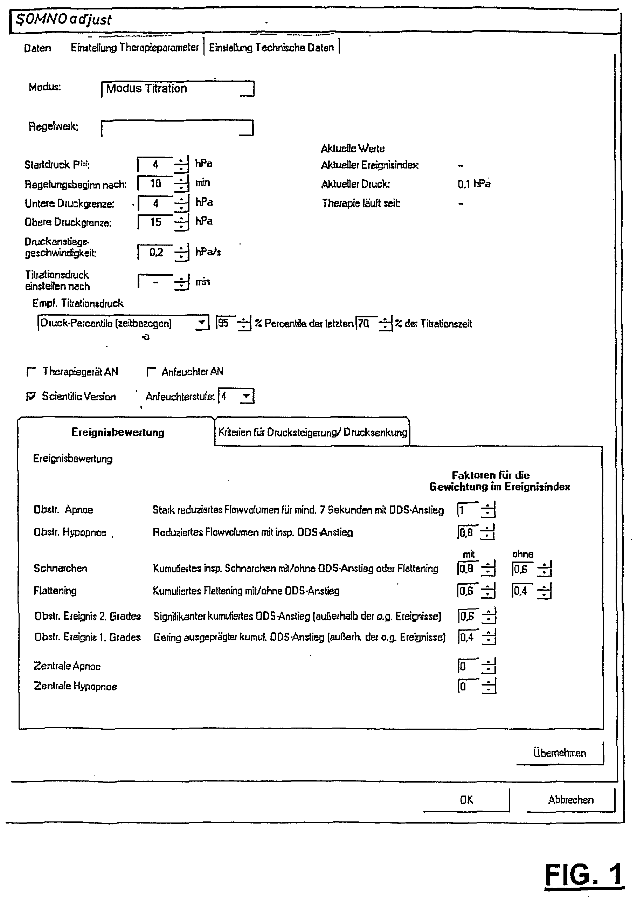

Der Startdruck kann gemäß Fig. 1 zwischen 3 -80 hPa in 0,1hPa-Schritten, der Regelungsbeginn zwischen 0 - 600 min in 5 min-Schritten, die untere Druckgrenze zwischen 3 - 80 hPa in 0,1hPa-Schritten, die obere Druckgrenze zwischen 3 - 80 hPa in 0,1hPa-Schritten, die Druckanstiegsgeschwindigkeit zwischen 0,1 - 0,6 hPa/s in 0,1 hPa/s-Schritten eingestellt werden. Dabei kann über die Software der aktuelle Ereignisindex [/h] und der aktuelle Druck angezeigt werden.

Ferner können in einer besonders bevorzugten Ausführungsform die Gewichtungsfaktoren und Regelkriterien für die Drucksteigerung und Drucksenkung zum Regelwerk zählen. Während softwareseitig alle Regelwerke speicherbar sind, ist im Therapiegerät nur ein benutzerdefiniertes Regelwerk und ein Default-Regelwerk gespeichert. Das Default-Regelwerk im Therapiegerät ist nicht überschreibbar. Änderungen der Einstellungen am Regelwerk im Therapiegerät können nur am benutzerdefinierten Regelwerk vorgenommen werden.

Im Modus Autotitration ist in besonders bevorzugter Form eine Split-Night-Option implementiert, d. h. die Titration wird nach einer definierten Zeit abgebrochen, der empfohlene Titrationsdruck berechnet und anschließend die Therapie mit dem ermittelten Druck durchgeführt. Wobei eine Mindesteinstellzeit nicht unterschritten werden kann und der Einstellbereich in Schritten erhöht werden kann (z.B. Einstellbereich 60 - 600 Minuten bzw. kein automatisches Beenden der Titration)

In einer bevorzugten Form wird der resultierende CPAP im Gerätekalender gespeichert und kann vom Arzt abgerufen werden. Die Bedienung am Gerät kann bspw. mittels Bedienerinterfaces, welches aus Tasten, drehbaren Einstellknöpfen, Touchscreens und/oder sonstigen Bedienelementen bestehen kann, vorgenommen werden; es können am Gerät nur die voreingestellten Druckverläufe und Regelwerke aktiviert, nicht editiert werden. Zum Editieren der Titrationsparameter, des Druckverlaufs oder zur manuellen Druckeinstellung wird über eine Schnittstelle die Verbindung mit einem PC und einer Software benötigt.

In einer besonders bevorzugten Form kann der empfohlener Titrationsdruck als zeitbezogene Druck-Percentile oder als ereignisbezogene Druck-Percentile berechnet werden. Dabei kann jeweils ein Wert für die Percentile und ein prozentualer Wert in definierten Schritten ab einem Grundwert (z.B. 10%) der Titrationszeit vorgegeben werden. Auch kann der Druck durch die Ausgabe des Enddruckes oder Median des letzten prozentualen Anteils der Titrationszeit in fest vorgegebenen oder wahlweise in variablen Einstellschritten (z.B. 5%) eingestellt bzw. bestimmt werden.

Es können für einen ausdruckbaren "Titrationsbericht" sowie in die Bildschirmanzeige der Startdruck, der Regelungsbeginn nach X Minuten, die untere Druckgrenze, die obere Druckgrenze, die Druckanstiegsgeschwindigkeit, der Titrationsdruck eingestellt nach X Minuten, die Therapiedauer und der empfohlene Titrationsdruck (Regel und Wert) aufgenommen werden.

Bei den Einstellungen der Druckgrenzen wird eine intelligente Veränderung angewandt: Bei Steigerung des Startdrucks über die untere und/oder obere Druckgrenze erfolgt eine automatische Steigerung der unteren und/oder oberen Druckgrenze auf den Startdruck. Bei Senkung des Startdrucks unter die untere Druckgrenze erfolgt eine automatische Senkung der unteren Druckgrenze auf den Startdruck. Bei Steigerung der unteren Druckgrenze über den Startdruck und/oder die obere Druckgrenze erfolgt eine automatische Steigerung des Startdrucks und/oder der oberen Druckgrenze auf die untere Druckgrenze. Bei Senkung der oberen Druckgrenze unter den Startdruck und/oder die untere Druckgrenze erfolgt eine automatische Senkung des Startdrucks und/oder der unteren Druckgrenze auf die obere Druckgrenze.

Bei den Einstellungen der Zeit für Regelungsbeginn und dem Split-Night-Zeitpunkt wird ebenfalls eine intelligente Veränderung angewandt: Bei Steigerung des Regelungsbeginns über die Zeit für den Beginn des Einstellen des Titrationsdruckes wird die Zeit für das Einstellen des Titrationsdruck automatisch angepasst.

Bei einer Senkung der Zeit für das Einstellen des Titrationsdruckes unter die Zeit für den Regelungsbeginn wird die Zeit für den Regelungsbeginn automatisch auf den gewählten Betrag abgesenkt.

Wird während der Autotitration die Nasalmaske abgenommen (z. B. Patient geht zur Toilette) so wird die Ereignisauswertung, Indexbildung und Druckanpassung angehalten, die Datenspeicherung läuft weiter. Im Display wird ein Hinweis angezeigt (z. B: "PAUSE"). Das Gebläse wird deaktiviert (oder mit minimaler Drehzahl betrieben). Bei einer Unterbrechungsdauer unter einer einstellbaren oder festen Zeitgrenze wird die Autotitration (Ereigniserkennung und Druckanpassung) anschließend wieder fortgesetzt, bei längerer Unterbrechung wird das Gerät ausgeschaltet.

Die Indexbildung wird nach Unterbrechung unter einer einstellbaren oder festen Zeitgrenze neu initialisiert.

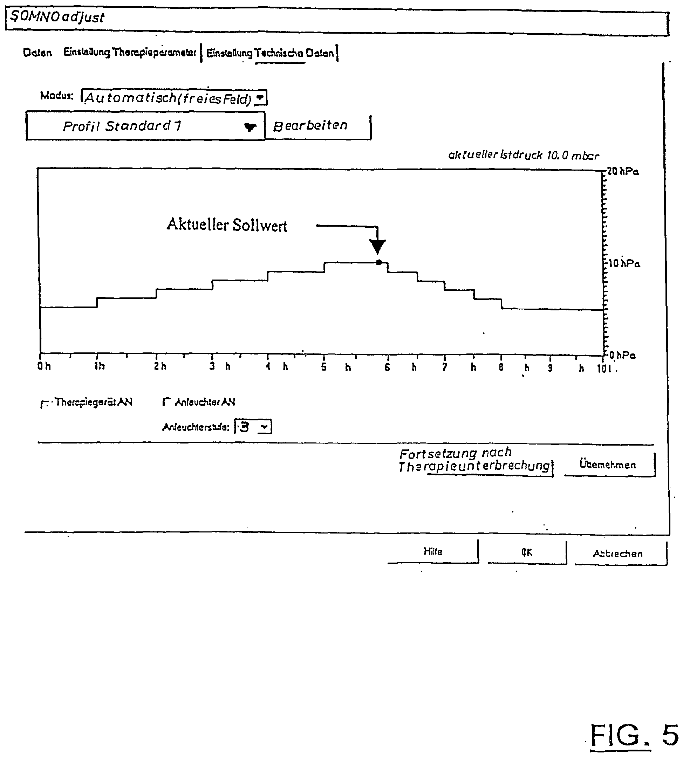

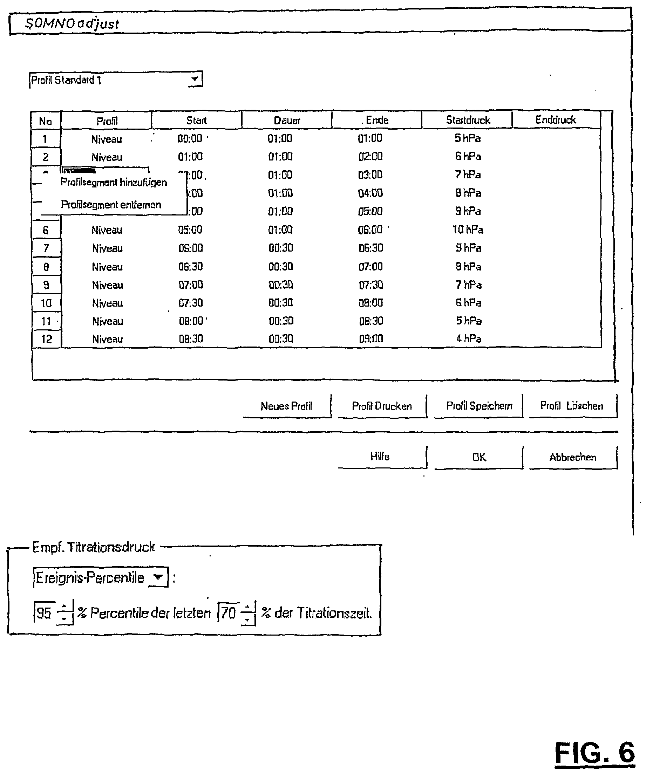

Im Modus Druckprofil kann die Druckanpassung nach dem gewählten Druckprofil durchgeführt werden. Ereignisse führen nicht zu einer Druckanpassung. Es sind feste und benutzerdefinierte Profile gespeichert. Diese gespeicherten Profile werden zum Beispiel in Fig.5 und Fig.6 deutlich.

Der Benutzer kann im Standby (Offline-) sowie im Online-Betrieb eines der beiden festen bzw. das benutzerdefinierte Druckprofil aktivieren. Die Druckprofile können im Standby (Offline-) sowie im Online-Betrieb mit einer PC-Software über die serielle Schnittstelle angezeigt und modifiziert (nur benutzerdefinierte Druckprofile) werden. Ist die Profildauer kürzer als die Therapiedauer, so wird die Therapie mit dem letzten Profildruck fortgeführt.

In diesem Modus kann die Titrationsdruckberechnung ebenfalls gewählt werden. Somit sind bei Therapiekontrollen mit auf den Patienten abgestimmten Profilen um den bisherigen Therapiedruckwert zur exakten Beurteilung der Drucksuffizienz möglich.

In einem Ausführungsbeispiel ist zur besseren Visualisierung und Nachvollziehen des aktuell anliegenden Druckes ein mitlaufender Marker (z.B. ein Punkt, Fadenkreus, etc.) auf dem Bildschirm der PC-Software und/oder auf dem Display des Bedienelementes zu sehen, welcher den aktuellen Solldruck und Position im Ablauf auf der Druckkurve über die Nacht darstellt.

Im Modus APAP erfolgt eine sofortige Druckanpassung aufgrund von therapierelevanten Einzelereignissen. Bei offener Maske und aktivierter Automatik schaltet sich das Gerät nach 5 Sekunden aus.

### APAP-Rest-Ereignisindex

Bisher wird bei jedem obstruktiven Ereignis der Druck angehoben, eine Weile konstant gehalten, und dann wieder langsam abgesenkt; dieses Prinzip ist so gut wie bei allen APAP-Geräte gleich.

Die Zeit, bei der der Ausgangsdruck (der zu Beginn des Ereignisses) wieder erreicht ist, wird als "Event reaction cycle time" (ERCT), bezeichnet so lange hat das Ereignis Einfluss auf den Druck.

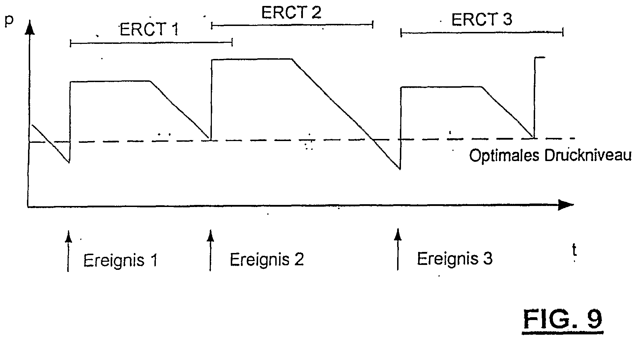

Wie in Fig. 9 zu sehen, folgt das nächste Ereignis schneller als die ERCT, so reagiert das Gerät wiederum, aber diesmal bei einem noch erhöhten Startdruck. Folgt das nächste Ereignis wiederum schneller als die ERCT des zweiten Ereignisses, wird der Druck noch höher angehoben etc. Es erfolgt also ein Netto-Druckanstieg.

Folgt das nächste Ereignis langsamer als die ERCT, ist der Druck schon tiefer als der Ausgangsdruck abgefallen. Es erfolgt nun zwar wieder eine Anhebung als Ereignisreaktion, aber auf einem niedrigeren Niveau als bei Ereignis 1. Folgt das nächste Ereignis wieder erst später als die ERCT, ist das Gerät auf einem noch niedrigeren Druckniveau, etc. Es gibt einen Netto-Druckabfall.

Folgt das nächste Ereignis genau nach Ablauf der ERCT, so ist das Gerät wieder genau auf dem Ausgangsdruck. Es pendelt also zwischen zwei Druckniveaus hin und her, der Druck bleibt netto konstant.

Durch geschickte Wahl der ERCT kann man also nun einen stabilen Arbeitspunkt "einregeln", also eine Zeit zwischen 2 Ereignissen, bei der das System stabil bleibt. Der Kehrwert der ERCT ist der sich in diesem stabilen Punkt ergebende Ereignisindex. Möchte man einen langfristig stabilen Rest-AHI von 5 unter der Therapie, muss man also die ERCT auf 12 min einstellen.

Da der Patient kein völlig stabiles, zeitlich unveränderliches System ist, ergibt eine entsprechende Druckregelung natürlich nicht genau einen AHI von 5, sie ist aber ein ziemlich robuster Ansatzpunkt zur Einstellung der Absenk-Wartezeiten und -Geschwindigkeit eines APAP-Gerätes.

Somit sind 4 Parameter einstellbar: Absenk-Wartezeiten und -Geschwindigkeit sowie der Druckanstieg bei Auftreten eines Ereignisses und die ERCT.

Die meisten APAP-Geräte senken viel zu früh ab und sind daher darauf angewiesen, dass der Patient mehrere leichte Obstruktionen entwickelt, auf die das Gerät schon wieder reagieren kann, bevor er wieder eine schwere, AHI-relevante Obstruktion entwickelt.

Da es aber keinerlei Wissen darüber gibt, ob der aktuelle 4 leichte Obstruktionen pro schwerer entwickelt oder 20 oder gar keine, ergibt sich mit den meisten APAP-Geräten ein sehr variabler AHI.

Der obige Ansatz dagegen "garantiert" (so lange der Patient stabil ist) einen AHI <= 5, selbst ohne irgendwelche leichten Obstruktionen.

Im Modus CPAP wird keine Druckanpassung durchgeführt. Das Gerät stellt konstant den eingestellten Druck zur Verfügung. Bei offener Maske und aktivierter Automatik schaltet sich das Gerät nach 5 Sekunden aus.

In einer besonders bevorzugten Form ist in dem Gerät ein wählbarer sogenannter Softstart-Modus implementiert, welcher bei Aktivierung des Gerätes die Drücke (hauptsächlich den CPAP-Druck) innerhalb eines bestimmten, einstellbaren Zeitraumes langsam automatisch auf das angestrebte Niveau (Modus CPAP) bzw. den angestrebten Druckbereich (Modus APAP) steigert. Diese Option kann im Autotitrationsmodus deaktiviert werden.

Im Modus Bi-Level wird keine Druckanpassung durchgeführt. Das Gerät stellt konstant den eingestellten in- und exspiratorischen Druck samt der dazugehörigen Beatmungsparameter (z.B. Geschwindigkeit des Druckwechsels, assistierte, kontrollierte, assistert/kontrollierte Beatmungsformen) zur Verfügung.

Im Modus Bi-Level Titration erfolgen Druck- und andere Parameteranpassungen in Abhängigkeit von einer Vielzahl aufgetretener respiratorischer Ereignisse. Dabei kann eine Volumen- oder druckkontrollierte Beatmung erfolgen. Bei offener Maske und aktivierter Automatik schaltet sich das Gerät nach 5 Sekunden aus.

In den Titrationsmodi können die bereits erwähnte Gewichtung von beim Atmen aufgetretenen Ereignissen und beatmungsdruckrelevante Artefakten, die für die Atemwegsstörung und ihrer Therapie relevant sind, und Artefaktsensibilitäten vom Anwender für die Beatmung mit Größen vorgegeben bzw. eingestellt werden.

Solche beatmungsdruckrelevante Artefakte sind zum Beispiel:
Aus MDS-, flow- und druckbezogenen Größen abgeleitete Parameter wie Lagewechsel des Patienten, Mund- und Maskenleckagen, Mundexspiration, Sprechen, Husten, Räuspern, Schlucken.

Zur Erkennung von Ereignissen und Artefakten werden u. a. folgende Signale verwendet:
Das (Geräte-) Flowsignal wird in einer weiteren besonders bevorzugten Ausführungsform aus der (Gebläse-) Drehzahl und dem aktuellen (Masken-) Druck ermittelt, diese Berechnung wird mit 100 Hz (10 ms) durchgeführt.
Das MDS wird durch Bandpass-Filterung (IIR-Filter) des MDS-Rohsignals erzeugt. Bei der Filterung werden in einer weiteren besonders bevorzugten Ausführungsform Frequenzen zwischen 19 - 21Hz durchgelassen, alle anderen Frequenzen werden unterdrückt.

Die Filterung wird mit 500Hz (2 ms) durchgeführt. Anschließend wird das Signal gleichgerichtet und über einen Zeitraum von 50ms das Maximum der Schwingung ermittelt. Nach 50 ms (25 Werten) wird das Maximum als neuer MDS-Wert gespeichert und das Maximum neu initialisiert.

Das Schnarchsignal wird durch Bandpass-Filterung (IIR-Filter) des MDS-Rohsignals erzeugt. Es werden in einer weiteren besonders bevorzugten Ausführungsform Frequenzen zwischen 65 - 190Hz durchgelassen, alle anderen Frequenzen werden unterdrückt.

Die Filterung wird mit 500Hz (2ms) durchgeführt. Anschließend wird das Signal gleichgerichtet.

ES soll gewährleistet werden, dass auch beim Auftreten von Störungen eine optimale Gerätesteuerung gewährleistet ist.

Diese Aufgabe wird dadurch gelöst, dass die Steuerung einen Analysator zur Erfassung mindestens eines Artefaktes aufweist und dass der Analysator derart mit der Steuerung gekoppelt ist, dass bei der Erkennung des Artefaktes eine von der Adaptionseinrichtung vorgegebene fehlerhafte Druckänderung vermieden wird.

Bei der Vorrichtung sowie dem Verfahren wird ausgenutzt, dass spezifische Artefakte zu typischen Beeinflussungen des messtechnisch erfassten Atemparameters führen. Die entsprechenden typischen zeitlichen Verläufe des Atemparameters, die einem bestimmten Artefakt zugeordnet sind, ermöglichen eine automatische Auswertung des Signalverlaufes hinsichtlich der jeweiligen Verläufe und somit eine steuerungstechnische Identifikation des jeweiligen Artefaktes.

Entsprechende spezifische und am Signalverlauf erkennbare Artefakte sind beispielsweise Mundexpiration, Mundatmung, Leckage, Schlucken, Sprechen oder Husten. Bei einer automatischen Erkennung derartiger Ereignisse ist es möglich, die Drucksteuerung derart zu modifizieren, dass diejenigen messtechnischen Parameter, die in einem Normalzustand von der Steuerung für eine Druckerhöhung oder eine Druckabsenkung ausgewertet werden und die bei einem Auftreten des jeweiligen Artefaktes nicht mehr zuverlässig ausgewertet werden können, für die Zeitdauer des Auftretens des Artefaktes von der Steuerung nicht berücksichtigt werden.

Ein prinzipieller Verfahrensablauf bei der Erkennung und steuerungstechnischen Berücksichtigung von Artefakten kann derart erfolgen, dass zunächst eine Erkennung von Inspirations- und Exspirationsphasen durch Auswertung eines mit dem Flow im Zusammenhang stehenden Signals oder durch Auswertung eines mit Druckschwankungen im Zusammenhang stehenden Signals durchgeführt wird. Es erfolgt eine Messung von Amplitude oder Leistung von exspiratorischen Druckschwankungen, die entweder vom Gerät erzeugt werden oder im Bereich des Patienten entstehen. Die vom Gerät erzeugten Druckschwankungen können durch ein moduliertes Drucksignal gebildet werden, beispielsweise einem MDS- oder FOT-Signal. Vom Patienten erzeugte Druckschwankungen können beispielsweise Zischgeräusche bei der Mundexspiration oder Leckagen sein.

Nach der Durchführung der Messung erfolgt ein Vergleich der gemessenen Amplitude oder der gemessenen Leistung mit einem aus vorangegangenen Exspirationsphasen oder Inspirationsphasen abgeleiteten Referenzwert. Bei einer Feststellung einer signifikanten Änderung erfolgt die Erkennung eines oder mehrerer Artefakttypen.

Optional ist es möglich, eine Unterscheidung der Artefakte anhand der Höhe der Änderung, anhand der Anzahl der konsekutiven Atemzüge mit Änderung oder durch eine Auswertung dahingehend vorzunehmen, ob die jeweilige Änderung nur in exspiratorischen oder auch in inspiratorischen Phasen im Vergleich zum vorangegangenen Atemzug auftritt.

Ebenfalls ist es optional möglich, eine zusätzliche Erhöhung der Analysequalität durch eine Auswertung des Flowverlaufs vorzunehmen, wenn beispielsweise die exspiratorische Halbwelle bei der Mundexspiration fehlt.

Es erfolgt ein Vergleich der exspiratorisch gemessenen Amplitude der Druckschwingung mit der inspiratorisch gemessenen Amplitude der Druckschwingung. Hierbei wird ein Vergleich der Mittelwerte der beiden Atemphasen durchgeführt.

Falls der expiratorische Wert um mindestens einen festgelegten ersten Betrag höher ist als der inspiratorische Wert, so erfolgt eine Erkennung eines einzelnen expiratorischen Artefaktes, beispielsweise Schlucken, Husten oder Sprechen.

Falls die expiratorischen Werte um mindestens einen festgelegten zweiten Betrag höher als die inspiratorischen Werte für eine vorher festgelegte Anzahl von Atemzügen sind, erfolgt eine Erkennung von Mundatmung oder Mundexpiration.

In Abhängigkeit vom Auswertungsergebnis erfolgt eine Unterdrückung der Auswertung des inspiratorischen Flowvolumens und somit eine Unterdrückung der Hypobnoeerkennung und ebenfalls eine Unterdrückung der Auswertung der inspiratorischen Amplitude der Druckschwingung im Zusammenhang mit der Oszilloresistometrie, da unter Mundatmung bzw. bei Auftreten eines expiratorischen Artefaktes eine entsprechende Reaktion unzuverlässig ist.

Gemäß einem anderen Ausführungsbeispiel erfolgt eine Messung der natürlichen Druckschwingungen in einem bestimmten Frequenzband durch Bandpassfilterung des gemessenen Drucksignals, um Schnarch- oder Zischgeräusche zu erkennen. Bei einem expiratorischen Anstieg erfolgt eine Erkennung von Mundexpirationen, bei einem inspiratorischen oder expiratorischen Anstieg die Erkennung einer Leckage. Es erfolgt auch in jedem Fall wieder eine Unterdrückung von Ereignissen bzw. eine Verschiebung von Schwellwerten, beispielsweise zur Schnarcherkennung.

Ein typischer Verfahrensablauf erfolgt dadurch, dass die Steuerung zur Durchführung einer APAP-Beatmung ausgebildet ist.

Gemäß einer Ausführungsform ist vorgesehen, dass der Analysator zur Auswertung eines Flowverlaufes ausgebildet ist.

Darüber hinaus ist auch daran gedacht, dass der Analysator zur Auswertung eines Druckverlaufes ausgebildet ist.

Eine Verfahrensvariante besteht darin, dass der Analysator zur Auswertung von Inspirationsphasen ausgebildet ist.

Darüber hinaus ist es auch möglich, dass der Analysator zur Auswertung von Exspirationsphasen ausgebildet ist.

Ein einfaches Auswertungsprinzip besteht darin, dass der Analysator zur Auswertung von Amplitudenwerten ausgebildet ist.

Darüber hinaus ist es auch möglich, dass der Analysator zur Auswertung von Leistungswerten ausgebildet ist.

Gemäß einer weiteren Ausführungsvariante ist vorgesehen, dass der Analysator einen Referenzwertkomperator aufweist.

Eine frequenzabhängige Signalauswertung wird dadurch unterstützt, dass eine Bandpassfilterung des gemessenen Drucksignals durchgeführt wird.

Insbesondere ist daran gedacht, dass ein Frequenzband bei der Bandpassfilterung derart definiert wird, dass eine Amplitude einer vom Gerät erzeugten Volumenschwingung gemessen wird.

Ein an die Bandpassfilterung angepasstes Anregungssignal kann dadurch bereitgestellt werden, dass die Volumenschwingung durch eine Membranpumpe erzeugt wird.

Gemäß einer anderen Ausführungsform ist es möglich, dass die Volumenschwingung durch eine Gebläseansteuerung erzeugt wird.

Ein guter Kompromiss zwischen einer einfachen gerätetechnischen Realisierbarkeit und einer guten Auswertbarkeit des Anregungssignals besteht darin, dass eine Volumenschwingung mit einer Frequenz von etwa 20 Hz erzeugt wird.

Gemäß einem typischen Auswertungsverfahren ist vorgesehen, dass eine Auswertung derart durchgeführt wird, dass durch einen exspiratorischen Anstieg der Druckschwingungsamplitude im Vergleich zu einem Referenzwert eine Erkennung einer exspiratorischen Verengung der Atemwege durchgeführt wird.

Eine besondere Variante der Störungserkennung besteht darin, dass eine Auswertung derart durchgeführt wird, dass einzelne oder kumulierte exspiratorische Verengungen als Artefakte bewertet werden.

Bei einer Auswertung der Häufigkeit des Auftretens von Artefakten ist es möglich, die Qualität des verwendeten Maskentyps zu beurteilen. Dies ermöglicht eine Qualitätskontrolle sowohl bei APAP- als auch bei CPAP- und Bilevel-Anwendungen.

Die oben bereits erwähnten Ereignisse können sein: Aus MDS-, flow- und druckbezogenen Größen abgeleitete Parameter wie obstruktive/zentrale Apnoen, obstruktive/zentrale Hypopnoen, Schnarchen mit und ohne Obstruktion der Atemwege, Flattening des Atemflusses, milde und schwere Obstruktionen, respiratorische Resistance und Compliance.

Die Ereignisaufzeichnung zur Therapiekontrolle beträgt z. B. 42 Tage.

In einer weiteren besonders bevorzugten Ausführungsform werden alle oder nur einzeln integrierte Ereignisse wie folgt erkannt:
Obstruktive Apnoe:
   Stark reduziertes Flowvolumen für mind. 7 Sekunden (und 2 Atemzüge) mit MDS-Anstieg. (MDS-Grenzwert steigt mit Druck; ab 12.5 hPa (Wert einstellbar) werden alle Apnoen als zentral gewertet; ab 7 hPa (Wert einstellbar) darf der MDS-Anstieg nicht erst am Ende der Apnoe erfolgen.)
Obstruktive Hypopnoe:
   Reduziertes Flowvolumen (2 konsekutive Inspirationen, max. 60% (Wert einstellbar) mit OE1 oder OE2 (In Abschnitten mit vielen zentr. Ereignissen: nur mit OE2)
Obstruktives Schnarchen:
   Kumuliertes Schnarchen (3 (Anzahl einstellbar) konsekutive Inspirationen) mit OE1, OE2 oder mit Flattening. Das Schnarchsignal überschreitet den definierten Schwellwert für eine definierte Mindestdauer (0,3s (Wert einstellbar)) während der Inspiration. Schnarchen wird in den Titrationsdaten (als Ereignis) und in der Wochencompliance (als Verlauf, Epoche mit/ohne Schnarchen) gespeichert.
Obstruktives Ereignis Grad 2:
   Signifikanter kumulierter (3 (Anzahl einstellbar) Atemzüge) inspiratorischer MDS-Anstieg
Obstruktives Flattening:
   Kumuliertes und deutlich ausgeprägtes Flattening (mind. 3 v. 6 Inspirationen (Anzahl einstellbar)), mindestens ein inspir. MDS-Anstieg
Primäres Schnarchen:
   Kumuliertes Schnarchen (3 (Anzahl einstellbar) konsekutive Inspirationen) ohne inspiratorischen MDS-Anstieg. Das Schnarchsignal überschreitet den definierten Schwellwert für eine definierte Mindestdauer (0,3s (Anzahl einstellbar)) während der Inspiration. Schnarchen wird in den Titrationsdaten (als Ereignis) und in der Wochencompliance (als Verlauf, Epoche mit/ohne Schnarchen) gespeichert.
Flattening:
   Kumuliertes Flattening (3 v. 6 (Anzahl einstellbar) Inspirationen)
Obstruktives Ereignis Grad 1:
   Gering ausgeprägter kumulierter (2 Atemzüge (Anzahl einstellbar)) inspiratorischer MDS-Anstieg
Zentrale Apnoe:
   Stark reduziertes Flowvolumen für mindestens 10 Sekunden ohne MDS-Anstieg.
Zentrale Hypopnoe:
   Reduziertes Flowvolumen (mind. 2 konsekutive Inspirationen und mind. 10 s (Anzahl und Mindestdauer einstellbar)) ohne MDS-Anstieg
Artefakt:
   Zeit- und Unruhekriterien z.B. bzgl. des Flow- und/oder MDS-Signals. Es werden keine Ereignisse mehr detektiert. Die Indexbildung geht unverändert weiter.
Maske offen:
   Solldruck kann nicht erreicht werden. Es werden keine Ereignisse mehr detektiert. Solldruck und Index werden eingefroren.
Mund- und Maskenleckage:
   Verlustflow über 0,33 l/s (= 20 l/min). Es werden keine Ereignisse mehr detektiert. Der Druck wird konstant gehalten bzw. entsprechend der Indexkriterien gesenkt, solange er über 10 hPa (Wert einstellbar) liegt.
Mundexspiration:
   Starke exspiratorische MDS-Anstiege (min. 3 x (Anzahl einstellbar)). Obstruktive Ereignisse und Hypopnoen werden nicht mehr detektiert. Die Indexbildung wird eingefroren.

### MDS-Artefakt

Exspiratorischer MDS-Anstieg. Obstruktive Ereignisse und Hypopnoen werden nicht mehr detektiert. Die Indexbildung wird unverändert fortgesetzt.

Weitere Anwendungen sind beatmungsparameterrelevante, respiratorische Ereignisse, wie zum Beispiel Arousals und Hyperventilation (insp. Volumen erhöht).

Zur besseren Erkennung und Abstimmung der Geräte individuell auf den Patienten können vom Anwender im Regelwerk der Titrationsmodi weiterhin festgelegt werden :
Ereignisindexgrenzwerte, die sich aus den oben genannten respiratorischen Ereignisse und deren Bewertung zusammensetzen und bei Über- bzw. Unterschreitung innerhalb des dem obig festgelegten Zeitraums zu einer Druckadaptation führen, absolute Druckanstiege des Geräts bzw. Druckabfälle bei Erreichen der vorherig festgelegten Ereignisindexgrenzwerte.

Für die Ereignisbewertung sind folgende Gewichtungsfaktoren definiert:
Gewichtungsfaktor Obstruktive Apnoe: Stark reduziertes Flowvolumen für mind. 7 Sekunden mit MDS-Anstieg: Einstellbereich: 0 - 1, in 0,1-Schritten
Gewichtungsfaktor Obstruktive Hypopnoe: Reduziertes Flowvolumen mit inspiratorischem MDS-Anstieg: Einstellbereich: 0 - 1, in 0,1-Schritten

Gewichtungsfaktor Obstruktives Ereignis 1. Grades: Signifikanter kumulierter MDS-Anstieg (außerhalb der o.g. Ereignisse):
Einstellbereich: 0 - 1, in 0,1-Schritten Gewichtungsfaktor Obstruktives Ereignis 2. Grades: Gering ausgeprägter kumulierter MDS-Anstieg (außerhalb der o.g. Ereignisse): Einstellbereich: 0 - 1, in 0,1-Schritten
Gewichtungsfaktor Schnarchen: Kumuliertes insp. Schnarchen mit/ohne MDS-Anstieg oder Flattening: Einstellbereich: 0 - 1, in 0,1-Schritten
Gewichtungsfaktor Flattening: Kumuliertes Flattening mit/ohne MDS-Anstieg: Einstellbereich: 0 - 1, in 0,1-Schritten
Gewichtungsfaktor Zentrale Apnoe/Hypopnoe: Einstellbereich: -1 bis 1, in 0,1-Schritten,
Die Druckanpassung nach Ereignissen wird entsprechend des gewählten Regelwerks durchgeführt. Der Ereignisindex wird nach Ablauf der Wartezeit für Drucksteigerung bzw. Drucksenkung aus den aufgetretenen Ereignissen während dieses Zeitraums berechnet. Es erfolgt eine Druckanhebung, wenn der Ereignisindex eine von zwei definierten Schwellen für Druckerhöhung überschreitet und die Wartezeit vor erneuter Druckerhöhung abgelaufen ist. Der Druck wird abgesenkt, wenn der Ereignisindex die definierte Schwelle für die Druckabsenkung unterschreitet und die Wartezeit für eine erneute Druckabsenkung abgelaufen ist. Nach einer Druckerhöhung oder - absenkung werden Ereignisindex und die Zähler für Zeitraum zur Berechnung des Index, sowie die Wartezeiten vor erneuter Druckanhebung / -absenkung auf 0 gesetzt.

Die Einstellbereiche Drucksteigerung können variieren, werden aber in bevorzugter Form wie folgt gewählt:
- Ereignisindex1: 0 ... 30 [/h], Auflösung: 0,1 [/h]
- Ereignisindex2: 0 ... 30 [/h], Auflösung: 0,1 [/h]
- Drucksteigerung1: 0 ... 10 [hPa], Auflösung 0,1 [hPa]
- Drucksteigerung2: 0 ... 10 [hPa], Auflösung 0,1 [hPa]
- Wartezeit (=Zeitraum für Indexberechnung): 1 ... 120 [min], Auflösung: 1 [min]

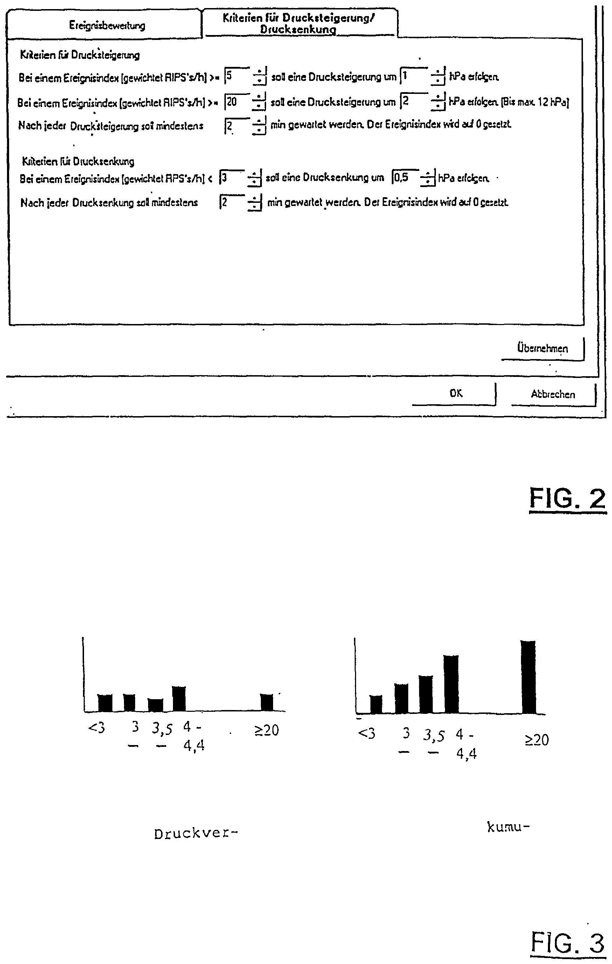

Um dem anfänglich starken Druckerhöhungsbedarf schnell entsprechen zu können, werden zwei Kriterien für die Drucksteigerung definiert, die Wartezeit beider Kriterien ist aber identisch zu halten. Fig. 2 zeigt eine entsprechende Bildschirmdarstellung.

Die Einstellbereiche Drucksenkung können variieren, werden aber in bevorzugter Form wie folgt gewählt:
- Ereignisindex: 0 ... 30 [/h], Auflösung: 0,1 [/h]
- Druckabsenkung: 0 ... 10 [hPa], Auflösung 0,1 [hPa]
- Wartezeit (=Zeitraum für Indexberechnung): 1 ... 120 [min], Auflösung: 1 [min],

Split-Night-Option, d. h. die Titration kann nach einer definierten Zeit abgebrochen werden. Ist die Split-Night-Option aktiviert, so wird nach Ablauf der angegebenen Dauer die Titration beendet. Es wird der empfohlene Titrationsdruck berechnet und eingestellt. Das Gerät wird anschließend solange mit diesem Druck betrieben bis der Benutzer das Gerät abschaltet.

Einstellbereiche:
kein Abbrechen der Titration
120 ... 600 [min], Auflösung: 5 [min] , (Default: keine Split-Night)

Während artefaktbehafteter Zeiträume (z.B. bei offener Maske) wird die Ereignisdetektion ausgesetzt, der Index wird eingefroren. Die Indexberechnung wird nach einem artefaktbehaftetem Zeitraum >= 5 Minuten (einstellbar) neu initialisiert.

### Titrationsdruckberechnung

Der empfohlene Titrationsdruck (im Modus Titration CPAP) kann, wenn eine Titrationsdauer von mindestens 2 Stunden (einstellbar) eingehalten wurde (inkl. Regelungsbeginnzeit), nach folgenden Verfahren ermittelt werden, dabei wird aus den applizierten Drücken nach den oben festgelegten Regeln zeit- , ereignis- und druckbezogene Werte für einen CPAP-Titrationsdruck abgeleitet.

### Druck-Percentile

Der Druckverlauf des angegebenen Zeitraums der Titration wird analysiert. Die Druck-Werte werden in 0,5 hPa-Klassen eingeteilt und der prozentuale Anteil (Häufigkeit) des Auftretens (der Druckklasse) im Analysezeitraum ermittelt. Anschließend werden die Klassen kumuliert und der Titrationsdruck entsprechend der angegebenen Percentile ermittelt (Kleinster Druckwert bei dem der prozentuale Anteil zum ersten Mal die angegebene Percentile übersteigt). Die Druckverläufe sind in Fig. 3 dargestellt.

Die Druck-Werte können in einer bevorzugten Form in folgende (36) Klassen eingeteilt werden:
- Klasse 0: p < 3 hPa
- Klasse 1: 3 <= p < 3,5 hPa
- Klasse 2: 3,5 <= p < 4 hPa
- ...
- Klasse 34: 19,5 <= p < 20 hPa
- Klasse 35: p <= 20 hPa

Der Analysezeitraum wird in % der Titrationszeit angegeben (letzte n% der Titrationszeit); Wertebereich: 10 ... 100%, Auflösung: 5%, (Default: 90% %). Die Percentile wird in Prozent angegeben; Wertebereich: 50 ... 100%, Auflösung: 5%, (Default: 95%).

### Ereignis-Percentile

Der Druckverlauf des angegebenen Analysezeitraums wird ausgewertet. Die Druckwerte bei Auftreten von obstruktiven Ereignissen (Obstr. , obstr. Apnoe, obstr. Hypopnoe, Schnarchen, Flattening) werden in bevorzugter Ausführungsform in 0,5 mbar - Klassen eingeteilt, die prozentualen Anteile ermittelt und aus den kumulierten Werten der Titrationsdruck ermittelt (Kleinster Druckwert bei dem der prozentuale Anteil zum ersten Mal die angegebene Percentile übersteigt). Die Einstellungen sind identisch zur Druck-Percentile.

### Enddruck

Bei der Einstellung des Enddruckes wird in bevorzugter Form der aktuelle (Soll-)Druck beim Erreichen der "Split-Night-Zeit" als Titrationsdruck verwendet bzw. als empfohlener Titrationsdruck gespeichert.

### Median

Der Druckverlauf des angegebenen Analysezeitraums wird in einer bevorzugten Ausführungsform ausgewertet und der "mittlere Wert" (Median) als Titrationsdruck zurückgeliefert.

Der Analysezeitraum wird in % der Titrationszeit angegeben (letzte n% der Titrationszeit) Wertebereich: 10 ... 100%, Auflösung: 5%, (Default: 90 %) Fehlererkennungen für Titrationsdruckberechnung:
Die Funktion zur Berechnung des Titrationsdruck liefert den ermittelten Titrationsdruck im Bereich 40 ... 200 (4 ... 20 mbar) zurück. Werte außerhalb dieses Bereichs sind als Fehlercodes reserviert.

In einer weiteren besonders bevorzugten Ausführungsform kann nach der Titrationsphase eine weitere Auswahlfunktion die nachgelagerte Modusauswahl tätigen.

In einer Ausführungsform kann sich die Auswahlfunktion an der Häufigkeit der aufgetretenen Ereignisse pro Stunde (z.B. dem AHI) oder/und auch der Variabilität der sich über die Diagnosephase einstellenden Druckwerte bei Ereignisauslösungen orientieren und eine Festlegung der Anfangsparameter auswählen.

Zudem kann bei gewählter Option die Therapieempfehlung direkt in die bedarfsabhängige Beatmung des Patienten ohne vorherige Kontrolle des Anwenders erfolgen. (Modus Automatisch)

### Druckanpassung im Modus Profil

Die Druckanpassung wird nach einem gewählten Druckprofil durchgeführt. Ereignisse führen nicht zu einer Druckanpassung.
- Das Druckprofil definiert den Druckverlauf im Modus Profil. Es können in bevorzugter Ausführungsform 3 Druckprofile gespeichert werden, wobei ein Druckprofil aus maximal 50 Segmenten bestehen kann und ein Segment jeweils die Zeit ab Gerätestart und den Druckwert enthalten kann. Diese können in den jeweiligen Bandbreiten geändert werden:
- Profil-ID: 1 ... 3 (1, 2: feste Profile, 3: benutzerdefiniertes Profil)
- Timer aktuelle Position im Druckprofil
- Anzahl der verwendeten Segmente: 1 ... 50
- Segmentdauer (Segment 1 ... 50): 0 ... 255 Minuten, Auflösung: 1 Minute
- Startdruck (Segment 1 ... 50): 2 ... 18 hPa, Auflösung: 0,1 hPa
- Enddruck (Segment 1 ... 50): 2 ... 18 hPa, Auflösung: 0,1 hPa

Als ein Ausführungsbeispiel kann die oben bereits erwähnte Therapieempfehlung die Art, innerhalb jeder Therapieform die Parameter sowie eine Empfehlung für Mensch-Maschine-Schnittstelle und Aerosoltherapie enthalten.

Dabei kann in besonders bevorzugter Form für die CPAP-Therapie der CPAP-Druck sowie die Steigung des Softstartes durch das Gerät empfohlen werden.

Dabei kann in besonders bevorzugter Form für die APAP-Therapie der Basisdruck (durchschnittlicher Druck) der letzten Therapienacht bzw. die Druckschwankungsbreite (obere/untere Druckgrenze) durch das Gerät empfohlen werden.

Dabei kann in besonders bevorzugter Form für die BiLevel-Therapie das inspiratorische, das exspiratorische Druckniveau und damit auch die Druckdifferenz, die In- und Exspirationszeit durch das Gerät empfohlen werden. Dabei kann eine Sicherheitsabfrage für ein minimales AMV erfolgen und die Parameter dahingehend angepasst werden, um den Patienten eine suffiziente Beatmung zu gewährleisten.

Das Gerät gibt nach Abschluss der Analysephase eine Aussage über die Eignung der für den Patienten zu verwendenden Maske, die sich aus bei verschiedenen Drücken aufgetretenen Ereignisse (v.a. Leckagen) ermittelt. Als Auswahl stehen mindestens die folgenden Arten: Nasal Pillow, Nasenmaske, Vollgesichtsmaske, Ganzkopfmaske und Tubus.

In einem weiteren Ausführungsbeispiel ist daran gedacht an Hand mindestens zwei verschiedener Parameter ausgewählt aus der Gruppe: Flow (Thermistor, Staudruck, Flowblende, aus Therapiegerät), Flowkontur, Effort (z.B. aus thorax- und abdomenexkursionserfassenden Sensoren), Atemwegswiderstand (MDS oder Ösophagussonde), Plethysmogramm, Sauerstoffsättigung, CO2, EKG, EEG, EMG (Kinn und bis zu 2 Bein-EMGs), EOG, Aktimeter, Mikrofon, Video, Körperlage, Licht, dem Gerät eine Zuordnung/Empfehlung für eine Beatmungsform (CPAP, APAP-, Bi-Level, weitere Beatmungsformen) erlauben zu können.

In einer Ausführungsform ist daran gedacht, die Messung modulierter Druck- und Flowwerte über verschiedenste Sensoren, wie Piezo, Thermo oder optisch, akkustisch, resistiv, kapazitiv, induktiv durchzuführen.

Da der Druck während der Exspirationsphase (EPAP) die Atemwege offen hält, wird in einer besonders bevorzugten Ausführungsform die Resistance über MDS gemessen und erfasst, ob die Lunge offen ist.

In einer besonders bevorzugten Ausführungsform wird MDS zur Erkennung von Atemtätigkeit und Atemphase z.B. über Aufweiten der oberen Atemwege etc. eingesetzt.

In einer besonders bevorzugten Ausführungsform wird aus MDS und der Compliance die Bi-level- Differenz, d.h. Ober - und Unterdruck bestimmt. (Modus Bi-Level Titration)

In einer besonders bevorzugten Ausführungsform ist daran gedacht das MDS zur Stimulation von Rezeptoren zu nutzen, um durch die Sensibilität des Pharynx ein Auslösen eines Reflexbogens zu provozieren, welches eine reflektorisch muskuläre Schienung der Atemwege zur Folge hat.

In einem weiteren Ausführungsbeispiel ist daran gedacht, die Atemarbeit aus dem MDS abzuleiten und über die Bestimmung der Impedanz eine Beurteilung der Atemanstrengungen herbeizuführen. Durch die Bestimmung des Atemantriebes oder der Atemanstrengungen können diese als Leitparameter für die Steuerung des Therapiegerätes herangezogen werden.

In einer bevorzugten Ausführungsform gibt das Gerät bei nicht ausreichender Therapie ein Meldung an den Arzt z.B. über bluetooth und Speichern dieser Meldung im Gerät.

Entsprechend der Diagnose des Arztes, kann das Gerät voreingestellt werden und erlaubt zusätzlich über den Anfeuchter, Vernebler, eine kontinuierliche Gabe/Zufuhr von Medikamenten, der sogenannten Aerosoltherapie , welche eine direkte Deposition von Medikamenten am Zielorgan bezweckt, d.h. in den tiefen Atemwegen. Eine bevorzugte Ausführungsform beschränkt sich auf die bronchialen Erkrankungen. Der Vorteil der Inhalation eines Medikamentes anstelle seiner Verabreichung als Tablette oder mittels einer Spritze besteht darin, dass die Substanz rasch den Wirkungsort erreicht und an anderen Organen weniger unerwünschte Nebenwirkungen entfaltet.

Folgende Arten der Erzeugung von Aerosolen stehen heute zur Verfügung: Die Druckluft- oder Ultraschallvernebler, die mit Treibgasen betriebenen Dosieraerosole und die Trockenpulverinhalatoren. Der Anfeuchter kann in allen Gerätemodi verwendet werden. Er wird durch Drücken der Anfeuchtertaste bzw. über die serielle Schnittstelle aktiviert bzw. deaktiviert. Die Anfeuchterstufe kann vom Benutzer per Tastatur oder serielle Schnittstelle eingestellt werden. Der Anfeuchter wird automatisch deaktiviert, sobald der Wasservorrat erschöpft ist oder der Anfeuchter vom Gerät getrennt wird. Dabei kann der Anfeuchter in mindestens sechs verschiedenen Anfeuchterstufen betrieben werden und bei entleertem Zustand eine Warnmeldung anzeigen.

Äußere Erscheinungsmerkmale und Ausführungsformen der Verbindungen, technische Rahmenbedingungen:
Die vorliegende Vorrichtung gestaltet sich äußerlich äußerst flexibel durch ihr modulare Zusammensetzung der Sensoren aus Sensorengruppen, die über Steckverbindungen, Kabel oder drahtlos miteinander, sowie mit einer Speicher- und einer Visualisierungseinheit verbunden werden, welche ein PC oder ein PDA sein kann; dabei ist die Konfigurierbarkeit der Messwerterfassungselektronik für verschiedene Sensoren (z. B. andere Verstärkungsfaktoren, Filter, ...) beibehalten.

Als besonderer Vorteil kann die Konfiguration des Diagnosesystems vom PC oder PDA über Kabel (direkt oder über Netzwerk) oder drahtlos durchgeführt werden; die Bedienung ist am Gerät selbst mit einem Bedienerinterface möglich.

In bevorzugten Ausführungsform ist die Speicherung der Daten im Gerät zur nachträglichen Auswertung z. B. am PC möglich.

In einer weiteren Ausführung ist das Therapiegerät mit einem Speicher versehen, der einfach entnommen werden (z. B. Compact Flash Karte) oder von Ferne ausgelesen werden kann (über Mobilfunk, Internet, Telefonleitung, ...).

Die Stromversorgung ist über Batterie oder Akku möglich. Es wird ein Selbsttest zumindest nach Einschalten, sowie eine wiederholte Kontrolle des Batterieladezustands durchgeführt.

Des Weiteren stehen freie Kanäle zur analogen Einspeisung weiterer Signale mit oder ohne Verstärkung zur Verfügung.

In einer besonderen Ausführungsform wird am Gerät zudem die Güte einzelner Signale, z. B. durch Impedanzmessung von Elektroden, über LEDs oder ein Display angezeigt.

In einer weiteren Ausführungsform ist daran gedacht, eine Einweg-EEG-Elektrode und eine zugehörige Verstärkerschaltung mit einem A-/D-Wandler und einem Algorithmus, der (evtl. mittels Neuronaler Netze und Fuzzy-Logik, Kybernetik) Schlafstadien erkennt, zu kombinieren, um auf die einzelnen Schlafstadien mit gezielten Parametereinstellungen reagieren zu können.

Zudem kann das Therapiegerät mit Speicher für Patientendaten (z. B. Gewicht, Geschlecht, Diagnose) ausgestattet sein.

Das Therapiegerät ist mit einer Identifizierungsnummer ausgestattet, mit der es sich in einem Netzwerk oder bei Verbindung mit einem PC eindeutig identifizieren kann, um ihm gespeicherte Informationen (über Wartung, Patient etc.) aus einer Datenbank zuzuordnen.

Das Therapiegerät ist mit einer Telemetrieeinheit für den ambulanten Einsatz ausgestattet, so dass die Diagnose bereits erstellt werden kann, bevor ein Serviceanbieter wieder zum Patient fährt und somit gleich die Therapie einleiten kann. Zudem kann das Gerät selbständig Kommunikation mit Servicestelle aufnehmen, z. B. zur Wartung oder im Fehlerfall oder zur Änderung der Konfiguration.

### Archivierung mehrerer Regelwerke

Beim softwarseitigen Einlesen der Therapieparameter aus dem Therapiegerät wird der Anwender aufgefordert das Regelwerk zu speichern, falls dieses mit keinem der softwareseitig gespeicherten Regelwerke übereinstimmt und sich das Therapiegerät im Modus Titration befindet. Änderungen am Regelwerk können softwareseitig gespeichert werden. Die Speicherung der Regelwerke erfolgt z. B. in einer XML-Datei im Verzeichnis, so dass die gespeicherten Regelwerke dem Gerät zugeführt, abgerufen und als Paramterliste ausgedruckt werden können.

Die Signalausgabe ist analog sowie digital möglich (z.B. für Polysomnographie).

Die Steuerung des Gerätes hat Zugriff auf die einzelnen Geräteeinheiten (Gebläse, Oszillationsgenerator, Anfeuchter, ...) und kann deren Parameter nach Bedarf ändern.

Die Datenspeicherung umfasst die Speicherung der Geräte-, Kalibrier- und Titrations-/Therapiedaten sowie aller sonstigen zur Funktion und zur Herleitung von Diagnosewerten nötigen Daten.

In einer weiteren besonders bevorzugten Ausführungsform ist die Kommunikation mit externen Geräten über eine serielle Schnittstelle, auch zur Bedienung des Geräts mit Ferneinstellung / PC und Datenexport zum PC möglich.

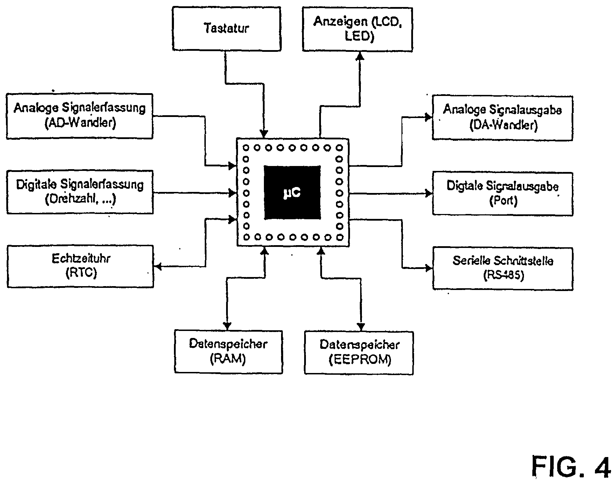

Als ein Ausführungsbeispiel kann die Hardware-Umgebung aus diversen Funktionsgruppen bestehen, die in Fig. 4 dargestellt sind.

Für die Anzeige ist ein mehrzeiliges Display vorgesehen. Es wird über das I2C-Interface des Mikrocontrollers angesprochen und enthält zusätzlich Symbole für z.B. Filterwechsel, Service, Anfeuchter, Anfeuchteralarm, Beatmungsparameter und -modus, Softstart, Druckanstieg, Druckabsenkung und Uhr sowie die Benennungen und Einheiten der dargestellten Werte.

Über das Gerätedisplay können Fehlermeldungen ausgegeben werden.

In einer weiteren bevorzugten Ausführungsform kann das MDS-Rohsignal mit 500Hz abgetastet werden und anschliessend eine Glättung durch (eine gleitende) Mittelwertbildung über mindestens 3 Werte erfolgen.

In einer weiteren bevorzugten Ausführungsform kann das Drucksignal mit 100Hz abgetastet werden und anschließend eine Glättung durch Mittelwertbildung über mindestens 100 Werte erfolgen. (Neuer Wert nach 1s)

In einer weiteren bevorzugten Ausführungsform kann die Anfeuchterspannung mit 50Hz abgetastet und anschließend eine Glättung durch Mittelwertbildung über mindestens 5 Werte erfolgen. (Neuer Wert nach 0,1s)

In einer weiteren bevorzugten Ausführungsform kann die (Geräte-)Temperatur mit 10Hz abgetastet werden und anschließend eine Glättung durch Mittelwertbildung über 10 Werte (Neuer Wert nach 1s) erfolgen.

In einer weiteren bevorzugten Ausführungsform kann die Gebläsedrehzahl über ein im Powerboard erzeugtes Signal erfasst werden. Mit einer Timer-Einheit (Capture-Funktion) des Mikrocontrollers wird die Laufzeit der Impulse gemessen und in eine Drehzahl umgerechnet.

In einer weiteren bevorzugten Ausführungsform kann die Drehzahl des Oszillationsgenerators (Pumpe) durch Auswerten des Mikrofonsignals bestimmt werden. Mit einer Timereinheit des Mikrocontrollers werden die Mikrofonimpulse (2 pro Umdrehung) über einen Zeitraum von 500ms erfasst und daraus die aktuelle Drehzahl des Oszillationsgenerators (Pumpe) berechnet.

In einer weiteren bevorzugten Ausführungsform kann für die analoge Signalausgabe ein 4-Kanal-DA-Wandler mit einer Auflösung von 10-Bit verwendet werden. Zwei Kanäle des DA-Wandlers werden für interne Zwecke verwendet (Sollwert Gebläsedrehzahl). Die beiden anderen Kanäle dienen zur Ausgabe von Signalen an externe Geräte (z. B. Polysomnograph). Über die serielle Schnittstelle kann mit der geräteunterstützenden Software das auszugebende Signal festgelegt werden (Modifikation der entsprechenden EEPROM-Zelle). Die Einstellungen werden im EEPROM gespeichert. Am Gerät können geräte- und patientenbezogene Signale für die Ausgabe gewählt werden, wie z.B. für die Ausgabe gewählt werden: MDS, Druck, Flow, Verlustflow

In einer bevorzugten Ausführungsform kann in der Standardeinstellung das MDS- und das Flowsignal ausgegeben werden.

In einer weiteren, bevorzugten Ausführungsform wird Anfeuchterleistung über eine PWM (Pulsweitenmodulation) gesteuert. Die PWM wird im Mikrocontroller (mit einer Capture/Compare-Einheit) erzeugt und (über einen Portpin) an das Powerboard weitergegeben.

In einer weiteren, bevorzugten Ausführungsform wird das Gebläse über einen Portpin freigeschaltet oder gesperrt.

In einer weiteren, bevorzugten Ausführungsform wird die Drehzahl des Oszillationsgenerators (Pumpe) über eine PWM geregelt. Die PWM wird im Mikrocontroller (mit einer Capture/Compare-Einheit) erzeugt und (über einen Portpin) ausgegeben.

In einer weiteren, bevorzugten Ausführungsform wird die externe Echtzeituhr über das I2C-Interface des Mikrocontrollers angesprochen. Die Einstellung von Datum und Uhrzeit (über die serielle Schnittstelle bzw. Tastatur) ist nur im Standby (Offline) möglich.

In einer weiteren, bevorzugten Ausführungsform kann der Datenspeicher des Gerätes im Modus APAP mindestens untere Druckgrenze, obere Druckgrenze, Druckanstiegsgeschwindigkeit, Softstartdruck, Softstartzeit und im Modus CPAP den CPAP, Softstartdruck und die Softstartzeit speichern.

Die Aufzeichnungszeit für die Daten der letzten Nacht beträgt ≥ 8 Stunden. Zudem werden die Kalibrierdaten des Drucksensors gespeichert.

In einer weiteren bevorzugten Ausführungsform wird die Jahrescompliance über den Zeitraum von 366 Tagen aufgezeichnet. Gespeichert wird die Anwendungszeit (Gerät eingeschaltet, Druck > Schwelle) pro Tag in 0,1h-Schritten. Ein Tag ist mit 24 Stunden definiert, wobei der Tageswechsel um 12.00 mittags erfolgt.

In einer bevorzugten Ausführungsform wird die Anfeuchtercompliance über den Zeitraum von 366 Tagen aufgezeichnet. Gespeichert wird die Anwendungszeit (Betrieb mit Anfeuchter) pro Tag in 0,1h-Schritten. Ein Tag ist mit 24 Stunden definiert, wobei der Tageswechsel um 12.00 mittags erfolgt.

In einer bevorzugten Ausführungsform werden die Titrationsdaten über einen Zeitraum von maximal 10 Stunden aufgezeichnet. Es werden maximal 10 Aufzeichnungen (Therapiestarts) gespeichert. Werden mehr als 10 Aufzeichungen durchgeführt oder wird die maximale Aufzeichnungszeit überschritten, so werden die ältesten Einträge bzw. Daten überschrieben. Aufzeichnungen mit einer Dauer < 5 Minuten werden beim nächsten Gerätestart überschrieben.

In einer weiteren bevorzugten Ausführungsform wird die Wochencompliance über einen Zeitraum von 30 Tagen aufgezeichnet. Pro Tag werden bis zu 30 Therapiestarts aufgezeichnet. Ein Tag ist mit 24 Stunden definiert wobei der Tageswechsel um 12.00 mittags erfolgt. Gespeichert werden nur Tage an denen eine Titration/Therapie durchgeführt wurde. Nach 30 Einträgen wird der älteste Eintrag überschrieben. Pro Eintrag werden folgende Daten aufgezeichnet:
Systemübergreifende Schnittstellen in einer besonders bevorzugten Ausführungsform
- Zur Kommunikation mit externen Geräten (PC, Ferneinstellung, Prüfrechner)
- Bluetooth
- Infrarot
- Speicherkarte
- Datenexport

Der Datenexport zum PC. Der PC (mit der Software) dient dabei als Master und fordert vom Gerät die Daten an. Das Gerät sendet die geforderten Daten, wobei am Ende der Übertragung eine Checksumme gesendet wird.

Die Übertragungszeit für Titrations- und Wochencompliance kann (zusammen) 1 Minute nicht überschreiten.

Über die serielle Schnittstelle(RS485) können mittels eines Prüfrechners während der Prüfung Geräteparameter abgefragt / modifiziert werden.

In einem anderen Regelwerk für die Autotitration CPAP wird bei auftretenden Arousals der Druck schneller erhöht. Die untere Druckgrenze wird bei Therapiekontrollen unterhalb der bisher beim Patienten eingestellten gewählt, um die Notwendigkeit des höheren Druckniveaus zu testen. Dies kann auch über ein entsprechend eingestelltes Druckprofil erfolgen.

In einem weiteren Ausführungsbeispiel erfolgt die Druckregelung auch in Abhängigkeit des aktuellen oder bereits durchgemachen Schlafstadiums.

Insbesondere beginnt die Titration erst, wenn der Patient eingeschlafen ist und/oder es erfolgt eine Druckreduktion in nächtlichen Wachphasen des Patienten.

Als weitere bevorzugte Ausführungsform sind folgende Funktionen einzeln oder kombiniert zu nennen:
1. Die Autotitration CPAP oder Bi-level beginnt automatisch, sobald eine bestimmte Anzahl respiratorischer Störungen (z.B. 30 Apnoen) und/oder dadurch bedingter Arousals und/oder Sauerstoffentsättigungen und/oder andere durch schlafbezogene Atmungsstörungen bedingte pathologische Veränderungen aufgetreten sind.
2. Die Überdruckbeatmung wird mit einem höheren Druck eingeleitet, wenn der Patient Schwierigkeiten hat, einzuschlafen ("Empfindung des Patienten, nicht genügend Luft zu erhalten"). Gewöhnliche Konditionen verbunden mit diesem Problem sind nasale Stauungen, schwere Fettleibigkeit und vorherige chronische Behandlung mit CPAP.
   Wenn einige Versuche an einer langsamen Zunahme des Drucks an ein Niveau, welches zur Offenhaltung der Atemwege ausreichend ist, nicht toleriert werden, wird automatisch auf eine Autotitration Bi-level umgeschaltet.
3.Wenn eine andauernde arterielle Sauerstoffentsättigung (SaO2 < 88%) trotz ausreichenden Luftstroms bei CPAP auftritt, wird zuerst der Versuch einer Zunahme des CPAP von 1-2 cmH2O angestrebt. Wenn hohe Niveaus von CPAP bereits benutzt werden, wird die Bilevel-Beatmung versucht. Wenn diese Versuche erfolglos sind, wird zusätzlicher Sauerstoff dem System in 1-LPM-Stufen bis SaO2 > 90% hinzugefügt.
4.Wenn der Patient Schwierigkeiten hat, die nasale Beatmung begleitet von einer nasalen Verengung zuzulassen, wird automatisch ein Anfeuchter hinzugeschaltet. Falls die Schwierigkeiten weiterhin bestehen, wird die Meldung zur Gabe eines Vasokonstrikors (gefäßverengendes Mittel) an den Arzt ausgegeben. Andernfalls wird die Empfehlung zur Titration mit einer Vollgesichtsmaske ausgegeben.
5.Wenn Patienten ein Level des CPAP- oder Bilevel-Druckes, welcher aussreicht, um die Atemwege zu stabilisieren, nicht tolerieren, so wird ein Hinweis oder über eine Schnittstelle die Anhebung des Bettkopfes um 30° ausgegeben bzw. durchgeführt oder eine Empfehlung zur seitlichen Lagerung des Patienten ausgegeben.
6. Wenn hohe Mundleckagen auftreten, wird der Anfeuchter angeschaltet und die Empfehlung zur Anwendung eines Kinnbügel ausgegeben. Wenn dieses erfolglos bleibt, wird auf Bilevel-Beatmung oder APAP umgeschaltet, um den Mitteldruck zu senken. Wenn dieses nicht erfolgreich ist, wird die Empfehlung zum Anlegen einer Vollgesichtsmaske angezeigt.
7. Bi-Leveltitration wird wie folgt eingestellt: IPAP = EPAP werden erhöht, bis obstruktive Apnoe nicht mehr vorhanden sind. Dann erhöhte sich IPAP, bis Hypopnea/Snoring/Sauerstoffentsättigung abgeschaffen sind. Falls Ereignisse auftreten trotz maximal, toleriertem IPAP, wird EPAP in 1cmH2O Schritten erhöht, bis diese nicht mehr auftreten und dennoch eine ausreichende Ventilation vorhanden sind.

Sauerstoff-Titrierung:
1. Wenn der Patient Sauerstoff benutzt, um eine ausreichende arterielle Sauerstoffsättigung während des Tages beizubehalten, wird zusätzlicher Sauerstoff mit der üblichen Strömungsgeschwindigkeit auf den Anfang der Diagnosestudie eingeleitet, es sei denn, es ist anders durch den Anwender eingestellt.
2. Wenn arterielle Sauerstoffentsättignung in Abwesenheit von Apnoen, Hypopnoen oder schweres Schnarchen anhält, wird eine Sauerstofftitrierung eingeleitet - aber nur nach einer verwendbaren Diagnoseperiode, um die Notwendigkeit der Sauerstoffgabe zu dokumentieren. Wenn das schwere Schnarchen zusammen mit einer Sauerstoffentsättigung auftritt, wird eine CPAP-Behandlung versucht, bevor Sauerstoff eingeleitet wird.
3. Wenn Sauerstoff eine Beatmungsbehandlung hinzugefügt wird oder eine Sauerstofftitrierung aufgrund einer Sauerstoffentsättignung druchgeführt wird, die nicht mit Apnoe/Hypopnea zusammenhängt, wird Sauerstoff in den 1-LPM-Stufensprüngen aufwärts titriert, bis die arterielle Sauerstoffsättigung > 92% ist (> 90%, wenn grösser als 3 LPM angefordert wird). Es gibt ein Maximumswert (z.B. 5LPM), der automatisch ohne Arztberatung von seiten des Gerätes eingestellt werden kann, höhere Werte können manuell eingestellt werden.

Zentrales Apnoe:
1. Wenn eine obstruktive oder gemischte Apnoe in eine zentrale Apnoe vom Typ Cheyne-Stokes während der CPAP-Titrierung umschlägt, kann eine weitere ansteigende Titrierung des CPAP versucht werden. Wenn kein Druckniveau die zentrale Apnoe abschafft, wird das Druckniveau bis zu einem Niveau erhöht, das den obstruktiven Bestandteil abschafft oder 10-12 cmH2O - je nachdem, welches höher ist.
2. Kurze zentrale Apnoen in REM-Schlafphasen, die weder eine Sauerstoffentsättigung noch eine Arousal zur Folge haben, werden nicht mit einer weiteren Anhebung des CPAP durch das Gerät behandelt.
3. Wenn häufige zentrale Apnoen (nicht vom Typ Cheyne-Stokes) und darauf folgende Arousals während einer CPAP-Titrierung vom Gerät erkannt werden, wird nach vorherigem Nachweis von Schnarchen/Flattening eine höheres Niveaus von CPAP angewendet. Wenn dieses nicht erfolgreich ist, wird ein etwas geringeres CPAP-Niveau versucht (mögliche Arousals neben erhöhten Druck/Leckagen) .

Als weitere bevorzugte Ausführungsform sind folgende Funktionen einzeln oder kombiniert zu nennen:
Die Titration beginnt gemäß Fig. 1 mit einem vorgegebenen Startdruck pini (1). Druckober(4)- und - untergrenzen (3) werden vorgegeben. Eine Druckanstiegsgeschwindigkeit (5) wird ebenfalls vorgegeben. Zudem ist die Zeit, nach der die Regelung beginnt und endet, vom Anwender einstellbar (2).

Fig. 6 zeigt eine Darstellung verschiedener, im Profilmodus vorzunehmender Einstellungen. Über diese Eingabemaske kann die Druckabfolge mit einzelnen Drucksequenzen in zeitlicher Länge, Beginn sowie appliziertem Druck eingestellt werden. Somit hat der Anwender die Möglichkeit, individuell für jeden Patienten die Druckverläufe einzustellen und als Profil abzuspeichern

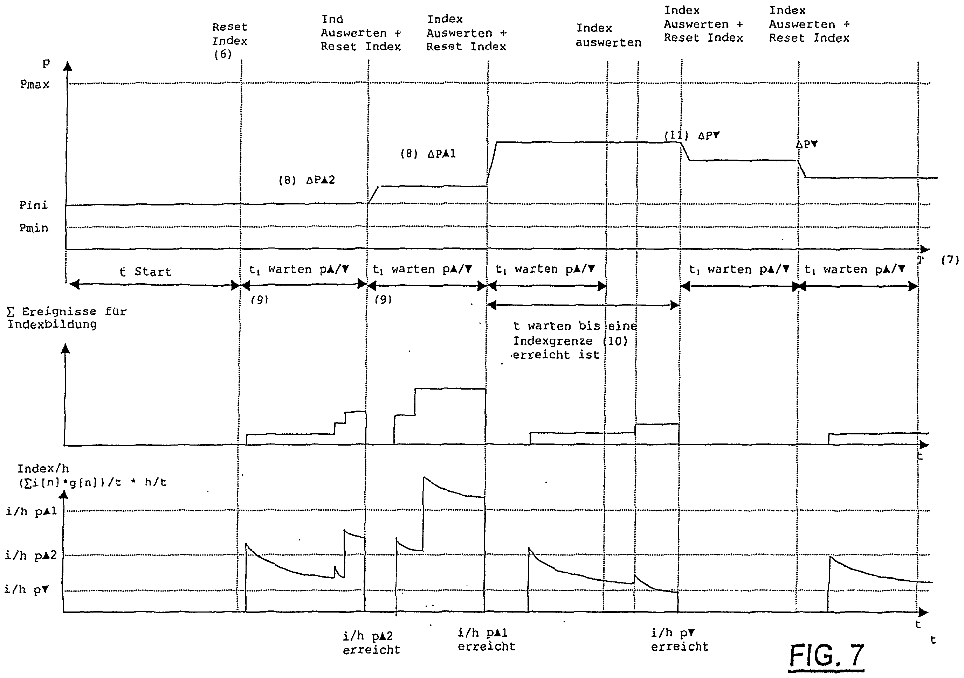

Gemäß Fig. 7 werden abhängig von den gewichteten Ereignisindices (6) über einen Gesamtzeitraum t (7) unterschiedliche Drücke (8) während der Zeit ti (9) dem Patienten zugeführt. Druckanstiegsgeschwindigkeit (5) und Minimalwerte für die Zeit ti (9) sind einstellbar. Während ti bleibt der applizierte Druck konstant, der aktuelle Ereignisindex wird nach jeder erforderlichen Druckanpassung zurückgesetzt und während ti laufend neu berechnet. Ist ein Ereignisindexgrenzwert (10) erreicht, erfolgt eine Druckanpassung (11).

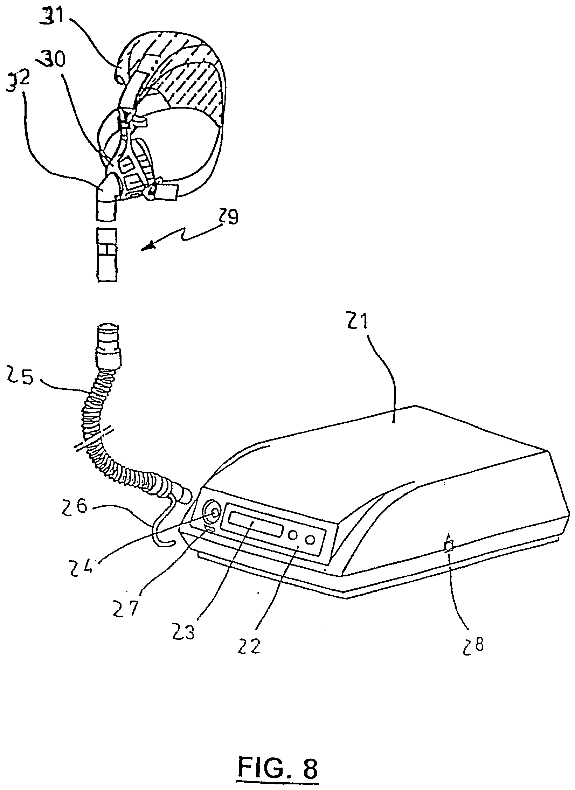

Fig. 8 zeigt den grundsätzlichen Aufbau einer Vorrichtung zur Beatmung. Im Bereich eines Gerätegehäuses (21) mit Bedienfeld (22) sowie Anzeige (23) ist in einem Geräteinnenraum eine Atemgaspumpe angeordnet. Über eine Kopplung (24) wird ein Verbindungsschlauch (25) angeschlossen. Entlang des Verbindungsschlauches (25) kann ein zusätzlicher Druckmeßschlauch (26) verlaufen, der über einen Druckeingangsstutzen (27) mit dem Gerätegehäuse (21) verbindbar ist. Zur Ermöglichung einer Datenübertragung weist das Gerätegehäuse (21) eine Schnittstelle (28) auf.

Im Bereich einer dem Gerätegehäuse (21) abgewandten Ausdehnung des Verbindungsschlauches (25) ist ein Ausatmungselement (29) angeordnet. Ebenfalls kann ein Ausatemventil verwendet werden.

Darüber hinaus zeigt die Abbildung eine Beatmungsmaske (30), die als Nasalmaske ausgebildet ist. Eine Fixierung im Bereich eines Kopfes eines Patienten kann über eine Kopfhaube (31) erfolgen. Im Bereich ihrer dem Verbindungsschlauch (25) zugewandten Ausdehnung weist die Beatmungsmaske (30) ein Kupplungselement (32) auf.

## Patentansprüche

1. Vorrichtung zur Beatmung, die eine mit einem Patienteninterface verbindbare Atemgasquelle sowie eine Steuereinrichtung aufweist, die zur Vorgabe von mindestens zwei unterschiedlichen Modi der Beatmung ausgebildet ist und bei der die Steuereinrichtung zur automatischen Ermittlung, Auswahl und Anwendung der verschiedenen Modi der Beatmung bei wenigstens einigen Atemstörungen entsprechend einem aktuellen patientenspezifischen Bedarf ausgebildet ist, **dadurch gekennzeichnet, dass** die Vorrichtung mit einer Identifizierungsnummer ausgestattet ist, mit der in einem Netzwerk oder bei Verbindung mit einem PC eine eindeutige Identifizierung der Vorrichtung erfolgt, um der Vorrichtung gespeicherte Informationen aus einer Datenbank zuzuordnen und dass die Vorrichtung mit einer Telemetrieeinheit für den ambulanten Einsatz ausgestattet ist und wobei der patientenspezifische Bedarf unter Verwendung mindestens eines Sensors zur Erfassung eines physiologischen und/oder gerätespezifischen Parameters und eines Analysators zur Signalauswertung ermittelt wird und wobei eine Adaptionseinrichtung zur Veränderung des Beatmungsmodus verwendet ist.

2. Vorrichtung nach Anspruch 1, **dadurch gekennzeichnet, dass** die Steuereinrichtung den Analysator zur Signalauswertung aufweist.

3. Vorrichtung nach einem der Ansprüche 1 oder 2, **dadurch gekennzeichnet, dass** zur Durchführung einer Druckanpassung ein Regelwerk verwendet ist.

4. Vorrichtung nach Anspruch 3, **dadurch gekennzeichnet, dass** das Regelwerk fest ausgebildet ist.

5. Vorrichtung nach Anspruch 3, **dadurch gekennzeichnet, dass** das Regelwerk benutzerdefiniert ausgebildet ist.

6. Vorrichtung nach einem der Ansprüche 1 bis 5, **dadurch gekennzeichnet, dass** die Steuereinrichtung die Adaptionseinrichtung zur Veränderung des Beatmungsmodus umfasst.

7. Vorrichtung nach einem der Ansprüche 1 bis 6, **dadurch gekennzeichnet, dass** die Steuereinrichtung eine Artefakterkennung umfasst.

8. Vorrichtung nach Anspruch 7, **dadurch gekennzeichnet, dass** der Analysator zur Unterdrückung einer Modusveränderung bei einer Artefakterkennung ausgebildet ist.

9. Vorrichtung nach einem der Ansprüche 1 bis 8, **dadurch gekennzeichnet, dass** der Analysator zur Erkennung von Inspirationsphasen und Exspirationsphasen ausgebildet ist.

10. Vorrichtung nach einem der Ansprüche 1 bis 9, **dadurch gekennzeichnet, dass** der Analysator zur Auswertung mindestens eines mit dem Flow im Zusammenhang stehenden Parameter ausgebildet ist.

11. Vorrichtung nach einem der Ansprüche 1 bis 9, **dadurch gekennzeichnet, dass** der Analysator zur Auswertung mindestens eines mit dem Druck im Zusammenhang stehenden Parameter ausgebildet ist.

12. Vorrichtung nach einem der Ansprüche 1 bis 11, **dadurch gekennzeichnet, dass** der Analysator eine Bandpaßfilterung aufweist.

13. Vorrichtung nach einem der Ansprüche 1 bis 12, **dadurch gekennzeichnet, dass** der Analysator eine Vergleichseinrichtung unter Berücksichtigung einer individuellen Patientenhistorie aufweist.

14. Vorrichtung nach einem der Ansprüche 1 bis 12, **dadurch gekennzeichnet, dass** der Analysator eine Vergleichseinrichtung unter Berücksichtigung voreingestellter Parameterwerte aufweist.

15. Vorrichtung nach einem der Ansprüche 1 bis 12, **dadurch gekennzeichnet, dass** der Analysator eine Vergleichseinrichtung unter Berücksichtigung von Idealwerten aufweist.

16. Vorrichtung nach einem der Ansprüche 1 bis 15, **dadurch gekennzeichnet, dass** die Steuereinrichtung zur Modusanpassung mit einem festgelegten Zeitintervall ausgebildet ist.

17. Vorrichtung nach einem der Ansprüche 1 bis 16, **dadurch gekennzeichnet, dass** die Steuereinrichtung zur Modusanpassung mit einem adaptiven Zeitintervall ausgebildet ist.

## Claims

1. Ventilation apparatus, comprising a respiratory gas source that is connectable to a patient interface and a control device embodied to specify at least two different ventilation modes, and in which the control device is embodied to automatically ascertain, select and use the various ventilation modes in accordance with the current patient-specific need in at least some respiratory disorders, **characterized in that** the apparatus is equipped with an identification number, by means of which there is a unique identification of the apparatus in a network or in the case of a connection to a PC in order to assign stored information from a database to the apparatus, and **in that** the apparatus is equipped with a telemetry unit for ambulatory use, and wherein the patient-specific need is ascertained using at least one sensor for capturing a physiological and/or appliance-specific parameter and an analyser for signal evaluation and wherein an adaptation device is used to alter the ventilation mode.

2. Apparatus according to Claim 1, **characterized in that** the control device comprises the analyser for signal evaluation.

3. Apparatus according to either of Claims 1 and 2, **characterized in that** guidelines are used to carry out the pressure adaptation.

4. Apparatus according to Claim 3, **characterized in that** the guidelines have a fixed embodiment.

5. Apparatus according to Claim 3, **characterized in that** the guidelines have a user-defined embodiment.

6. Apparatus according to any one of Claims 1 to 5, **characterized in that** the control device comprises the adaptation device for altering the ventilation mode.

7. Apparatus according to any one of Claims 1 to 6, **characterized in that** the control device comprises artefact detection.

8. Apparatus according to Claim 7, **characterized in that** the analyser is embodied to suppress a change of mode in the case of an artefact detection.

9. Apparatus according to any one of Claims 1 to 8, **characterized in that** the analyser is embodied to detect inspiration phases and expiration phases.

10. Apparatus according to any one of Claims 1 to 9, **characterized in that** the analyser is embodied to evaluate at least one parameter related to the flow.

11. Apparatus according to any one of Claims 1 to 9, **characterized in that** the analyser is embodied to evaluate at least one parameter related to the pressure.

12. Apparatus according to any one of Claims 1 to 11, **characterized in that** the analyser has bandpass filtering.

13. Apparatus according to any one of Claims 1 to 12, **characterized in that** the analyser comprises a comparison device, with an individual patient history being taken into account.

14. Apparatus according to any one of Claims 1 to 12, **characterized in that** the analyser comprises a comparison device, with pre-set parameter values being taken into account.

15. Apparatus according to any one of Claims 1 to 12, **characterized in that** the analyser comprises a comparison device, with ideal values being taken into account.

16. Apparatus according to any one of Claims 1 to 15, **characterized in that** the control device is embodied for mode adaptation with a defined time interval.

17. Apparatus according to any one of Claims 1 to 16, **characterized in that** the control device is embodied for mode adaptation with an adaptive time interval.

## Revendications

1. Dispositif de ventilation, présentant une source de gaz respiratoire pouvant être reliée à une interface patient ainsi qu'un dispositif de commande qui est réalisé pour spécifier au moins deux modes de ventilation différents, et dans lequel le dispositif de commande est réalisé pour déterminer, sélectionner et appliquer automatiquement les différents modes de ventilation dans le cas d'au moins certains troubles respiratoires selon un besoin actuel spécifique au patient, **caractérisé en ce que** le dispositif est équipé d'un numéro d'identification avec lequel une identification univoque du dispositif est effectuée dans un réseau ou lors d'une connexion à un PC afin d'associer au dispositif des informations mémorisées issues d'une base de données, et **en ce que** le dispositif est équipé d'une unité de télémétrie pour la mise en œuvre ambulatoire, et dans lequel le besoin spécifique au patient est déterminé en utilisant au moins un capteur pour acquérir un paramètre physiologique et/ou spécifique à l'appareil et d'un analyseur pour évaluer des signaux, et dans lequel un dispositif d'adaptation est utilisé pour changer le mode de ventilation.

2. Dispositif selon la revendication 1, **caractérisé en ce que** le dispositif de commande présente l'analyseur pour évaluer des signaux.

3. Dispositif selon l'une quelconque des revendications 1 et 2, **caractérisé en ce qu'**un ensemble de règles est utilisé pour effectuer une adaptation de la pression.

4. Dispositif selon la revendication 3, **caractérisé en ce que** l'ensemble de règles est réalisé de manière fixe.

5. Dispositif selon la revendication 3, **caractérisé en ce que** l'ensemble de règles est réalisé en étant défini par l'utilisateur.

6. Dispositif selon l'une quelconque des revendications 1 à 5, **caractérisé en ce que** le dispositif de commande comprend le dispositif d'adaptation pour changer le mode de ventilation.

7. Dispositif selon l'une quelconque des revendications 1 à 6, **caractérisé en ce que** le dispositif de commande comprend une reconnaissance des artéfacts.

8. Dispositif selon la revendication 7, **caractérisé en ce que** l'analyseur est réalisé pour supprimer un changement de mode si des artéfacts sont reconnus.

9. Dispositif selon l'une quelconque des revendications 1 à 8, **caractérisé en ce que** l'analyseur est réalisé pour reconnaître des phases d'inspiration et des phases d'expiration.

10. Dispositif selon l'une quelconque des revendications 1 à 9, **caractérisé en ce que** l'analyseur est réalisé pour évaluer au moins un paramètre en relation avec le flux.

11. Dispositif selon l'une quelconque des revendications 1 à 9, **caractérisé en ce que** l'analyseur est réalisé pour évaluer au moins un paramètre en relation avec la pression.

12. Dispositif selon l'une quelconque des revendications 1 à 11, **caractérisé en ce que** l'analyseur présente un filtrage passe-bas.

13. Dispositif selon l'une quelconque des revendications 1 à 12, **caractérisé en ce que** l'analyseur présente un comparateur en tenant compte d'un historique de patient individuel.

14. Dispositif selon l'une quelconque des revendications 1 à 12, **caractérisé en ce que** l'analyseur présente un comparateur en tenant compte de valeurs de paramètre préréglées.

15. Dispositif selon l'une quelconque des revendications 1 à 12, **caractérisé en ce que** l'analyseur présente un comparateur en tenant compte de valeurs idéales.

16. Dispositif selon l'une quelconque des revendications 1 à 15, **caractérisé en ce que** le dispositif de commande est réalisé pour adapter les modes avec un intervalle de temps fixe.

17. Dispositif selon l'une quelconque des revendications 1 à 16, **caractérisé en ce que** le dispositif de commande est réalisé pour adapter les modes avec un intervalle de temps adaptatif.
